# EUROPEAN PATENT SPECIFICATION

(11) **EP 3 387 135 B1**
(45) Date of publication and mention of the grant of the patent: **17.02.2021**
(21) Application number: 16829341.3
(22) Date of filing: 07.12.2016
(51) Int. Cl.: C12N 15/77

(54) **PROMOTERS FROM CORYNEBACTERIUM GLUTAMICUM**
PROMOTOREN AUS CORYNEBACTERIUM GLUTAMICUM
PROMOTEURS DE CORYNEBACTERIUM GLUTAMICUM

(30) Priority: 07.12.2015 US 201562264232 P
(43) Date of publication of application: 17.10.2018
(73) Proprietor: Zymergen Inc., Emeryville, CA 94608 (US)
(72) Inventor: SERBER, Zach, Sausalito, California 94965 (US); GORA, Katherine G., Oakland, California 94609 (US); MANCHESTER, Shawn P., Emeryville, CA 94608 (US)
(74) Representative: Cooley (UK) LLP
(86) International application number: PCT/US2016/065464
(87) International publication number: WO 2017/100376

(56) References cited:
- WO-A1-2006/028063
- DATABASE EMBL [Online] 20 September 2006 (2006-09-20), "DNA fragment having promoter function.", XP002767746, retrieved from EBI accession no. EM_PAT:DD324094 Database accession no. DD324094
- DATABASE Geneseq [Online] 8 March 2007 (2007-03-08), "Corynebacterium glutamicum DNA gyrase subunit B DNA, SEQ ID: 123.", XP002767747, retrieved from EBI accession no. GSN:AEM36105 Database accession no. AEM36105
- JOSE-LUIS ADRIO ET AL: "Recombinant organisms for production of industrial products", BIOENGINEERED BUGS, vol. 1, no. 2, 1 March 2010 (2010-03-01), pages 116-131, XP055141219, ISSN: 1949-1018, DOI: 10.4161/bbug.1.2.10484
- RASTEGARI H ET AL: "Improvement in the production of L-lysine by overexpression of aspartokinase (ASK) in C. glutamicum ATCC 21799", TROPICAL JOURNAL OF PHARMACEUTICAL RESEARCH 2013 PHARMACOTHERAPY GROUP NGA, vol. 12, no. 1, February 2013 (2013-02), pages 51-56, XP002767748, ISSN: 1596-5996
- DATABASE Geneseq [Online] 8 March 2007 (2007-03-08), "Corynebacterium glutamicum DNA gyrase subunit B DNA, SEQ ID: 123.", XP002770467, retrieved from EBI accession no. GSN:AEM36105 Database accession no. AEM36105

## Description

### BACKGROUND

### Field

The invention relates to native promoters comprising polynucleotides isolated from *Corynebacterium glutamicum,* and mutant promoters derived therefrom, host cells and recombinant vectors comprising the promoters, and methods of modifying the expression of target genes and producing biomolecules comprising culturing the host cells.

### Description of the Related Art

Strains of coryneform bacteria, in particular *Corynebacterium glutamicum,* play a significant role in the production of biomolecules such as amino acids, organic acids, vitamins, nucleosides and nucleotides, and continuous efforts are being made to improve production processes. Said processes may be improved with respect to fermentation related measures such as, for example, stirring and oxygen supply, or the composition of nutrient media, such as, for example, sugar concentration during fermentation, or the work-up into the product form, for example by means of ion exchange chromatography, or the intrinsic performance characteristics of the microorganism itself.

Performance characteristics can include, for example, yield, titer, productivity, by-product elimination, tolerance to process excursions, optimal growth temperature and growth rate. One way to improve performance of a microbial strain is to increase the expression of genes that control the production of a metabolite. Increasing expression of a gene can increase the activity of an enzyme that is encoded by that gene. Increasing enzyme activity can increase the rate of synthesis of the metabolic products made by the pathway to which that enzyme belongs. In some instances, increasing the rate of production of a metabolite can unbalance other cellular processes and inhibit growth of a microbial culture. Sometimes, down regulating activity is important to improve performance of a strain. For example, re-directing flux away from by-products can improve yield. Accordingly, fine-tuning of expression levels of the various components simultaneously within a metabolic pathway is often necessary.

Promoters regulate the rate at which genes are transcribed and can influence transcription in a variety of ways. Constitutive promoters, for example, direct the transcription of their associated genes at a constant rate regardless of the internal or external cellular conditions, while regulatable promoters increase or decrease the rate at which a gene is transcribed depending on the internal and/or the external cellular conditions, *e.g*. growth rate, temperature, responses to specific environmental chemicals, and the like. Promoters can be isolated from their normal cellular contexts and engineered to regulate the expression of virtually any gene, enabling the effective modification of cellular growth, product yield and/or other phenotypes of interest.

There is clearly a need for a broader assortment of well-defined *Corynebacterium* species promoters than has been heretofore described. Such promoters would be useful in the coordinated expression of genes in coryneform cells. For example, a collection of *C. glutamicum* promoters would facilitate the industrial-scale production of biomolecules in *C. glutamicum* cells by enhancing the expression of genes that encode components of the biosynthetic pathways for the desired biomolecules. The promoters described herein help meet these and other needs.

### BRIEF SUMMARY

In brief, the present disclosure is directed to native promoters comprising polynucleotides isolated from *Corynebacterium glutamicum,* and mutant promoters derived therefrom, which can each be encoded by short DNA sequences, ideally less than 100 base pairs, and which together represent a ladder of constitutive promoters having incrementally increasing expression levels. It is possible for various genes to be expressed advantageously under the control of said promoters.

One embodiment of the present invention relates to a promoter ladder comprising at least three promoter polynucleotide sequences selected from the group consisting of SEQ ID NOs: 1 to 8 , wherein said promoter ladder comprises a plurality of promoters with incrementally increasing levels of promoter activity.

One embodiment of the present invention relates to host cells comprising the promoter ladders described herein.

One embodiment of the present invention relates to methods of modifying the expression of one or more target genes, comprising culturing a host cell described herein, wherein the modification of each target gene is independently selected from: up-regulating and down-regulating. The target gene preferably codes for one or more polypeptides or proteins of the biosynthetic pathway of biomolecules including, *e.g.,* amino acids, organic acids, nucleic acids, proteins, and polymers.

Another embodiment of the present invention relates to methods of producing a biomolecule comprising culturing a host cell described herein, under conditions suitable for producing the biomolecule. In some embodiments the target gene is associated with a biosynthetic pathway producing a biomolecule selected from: amino acids, organic acids, flavors and fragrances, biofuels, proteins and enzymes, polymers/monomers and other biomaterials, lipids, nucleic acids, small molecule therapeutics, protein or peptide therapeutics, fine chemicals, and nutraceuticals. In preferred embodiments, the biomolecule is an L-amino acid. In specific embodiments, the L-amino acid is lysine.

In some embodiments, the host cell belongs to genus *Corynebacterium.* In some embodiments, the host cell is *Corynebacterium glutamicum.*

### BRIEF DESCRIPTION OF THE DRAWINGS

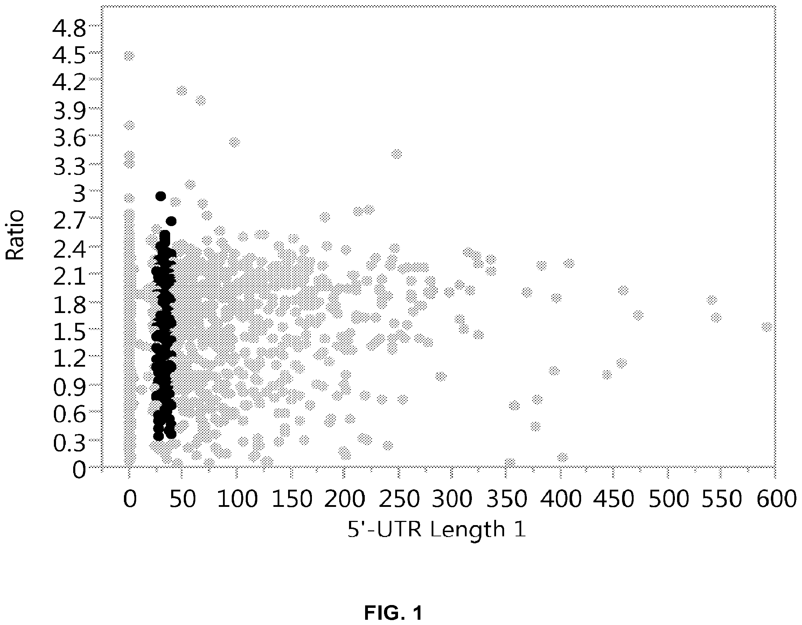

Figure 1 shows a graph of 5' UTR length (x axis) versus expression ratio across two growth conditions (y axis) for each gene in the *C. glutamicum* ATCC 13032 genome. Genes having both an expression ratio across the two growth conditions of between 0.33 and 3, and a 5' UTR length of between 26 and 40 base pairs are represented by black circles. Genes that failed to match both criteria are represented by grey circles.

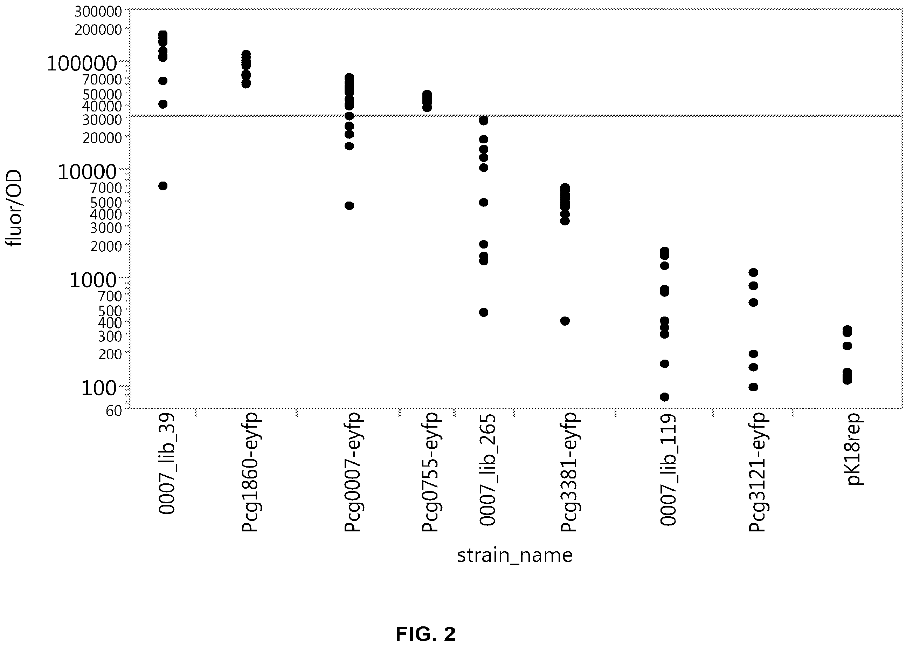

Figure 2 shows a graph of normalized activity (x axis) of eight candidate promoters (y axis) in a yellow fluorescent protein-based assay. Each biological replicate of each candidate promoter is represented by a black circle. The parent plasmid pK18rep acted as a negative control.

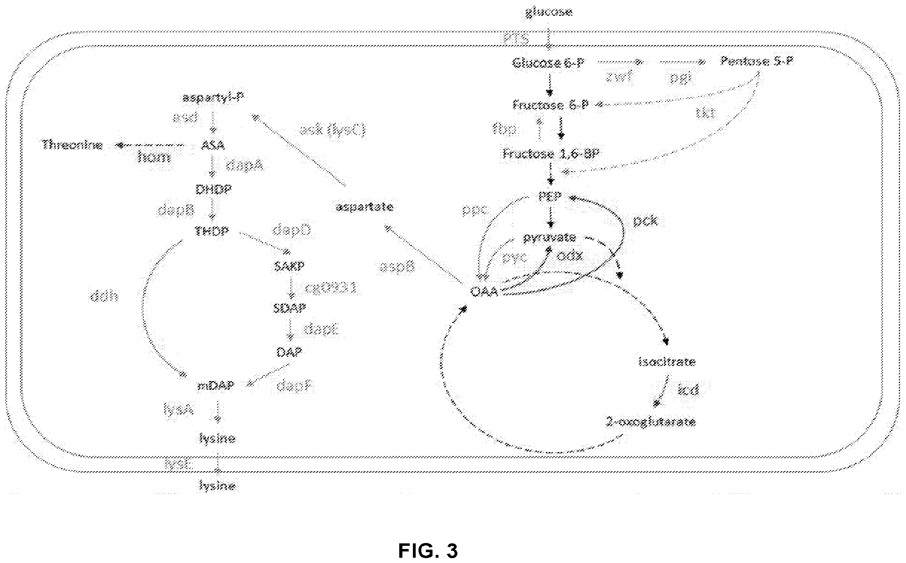

Figure 3 presents a diagram of the genetic and biochemical pathway for the biosynthesis of the amino acid L-lysine. Genes that divert intermediates in the biosynthetic pathway *(e.g., pck, odx, icd,* and *hom)* are underlined.

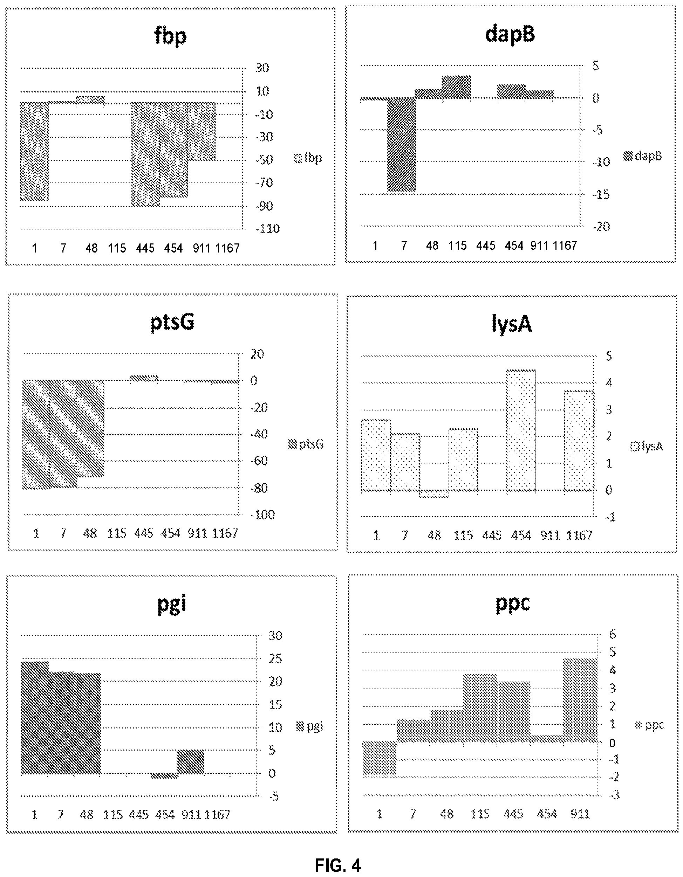

Figure 4 presents a graph of the results of exemplary embodiments according to the present specification of changes to L-lysine production in host cells of C. *glutamicum* transformed with recombinant nucleic acid molecules having promoter polynucleotide sequences selected from the group consisting of SEQ ID NOs:1 to 8 functionally linked to the heterologous target genes *fbp*, *dapB, ptsG, lysA, pgi,* and *ppc,* from *C. glutamicum.*

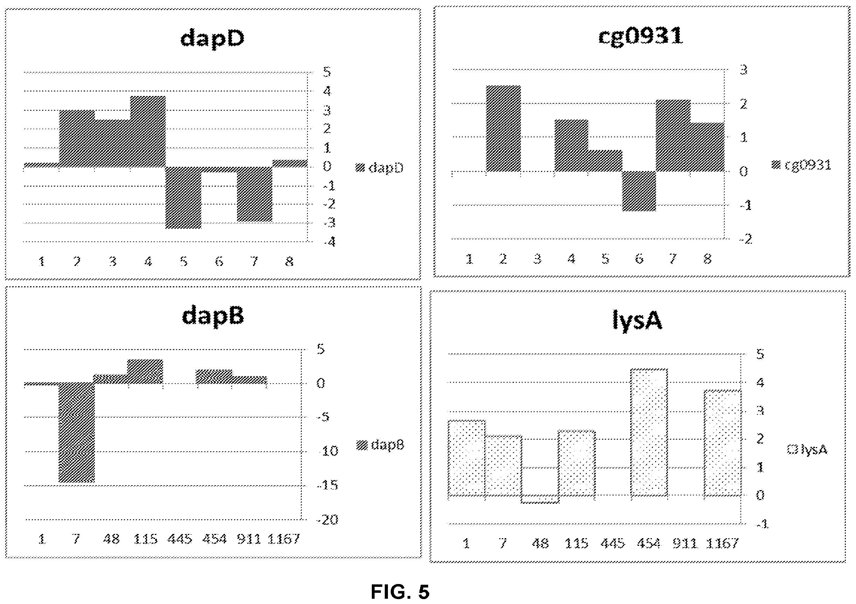

Figure 5 presents a graph of the results of exemplary embodiments according to the present specification of changes to L-lysine production in host cells of *C. glutamicum* transformed with recombinant nucleic acid molecules having promoter polynucleotide sequences selected from the group consisting of SEQ ID NOs:1 to 8 functionally linked to the heterologous target genes *dapS.* cg0931, DapB, and *lysA,* from *C. glutamicum.*

### DETAILED DESCRIPTION

In the following description, certain specific details are set forth in order to provide a thorough understanding of various embodiments of the disclosure. However, one skilled in the art will understand that the disclosure may be practiced without these details.

Unless the context requires otherwise, throughout the present specification and claims, the word "comprise" and variations thereof, such as, "comprises" and "comprising" are to be construed in an open, inclusive sense, that is as "including, but not limited to".

As used herein, the term "recombinant nucleic acid molecule" refers to a recombinant DNA molecule or a recombinant RNA molecule. A recombinant nucleic acid molecule is any nucleic acid molecule containing joined nucleic acid molecules from different original sources and not naturally attached together. Recombinant RNA molecules include RNA molecules transcribed from recombinant DNA molecules. In particular, a recombinant nucleic acid molecule includes a nucleic acid molecule comprising a promoter of SEQ ID NOs:1 to 8 functionally linked to a heterologous target gene.

As used herein, the term "heterologous target gene" refers to any gene or coding sequence that is not controlled in its natural state (*e.g.,* within a non-genetically modified cell) by the promoter to which it is operably linked in a particular genome. As provided herein, all target genes functionally linked to non-naturally occurring promoters are considered "heterologous target genes". More specifically, as promoter polynucleotide sequences of SEQ ID NOs:1, 5, and 7 do not occur in nature, all functionally linked target gene sequences are "heterologous target gene" sequences. As used herein, a heterologous target gene can include one or more target genes that are part of an operon. That is, the endogenous promoter of an operon is replaced with a promoter polynucleotide sequence having a nucleic sequence of SEQ ID NOs: 1 to 8. As used herein, the term "promoter polynucleotide sequence" refers to nucleic acids having a sequence as recited in the associated SEQ ID NO.

Reference throughout this specification to "one embodiment" or "an embodiment" means that a particular feature, structure or characteristic described in connection with the embodiment is included in at least one embodiment of the present disclosure. Thus, the appearances of the phrases "in one embodiment" or "in an embodiment" in various places throughout this specification are not necessarily all referring to the same embodiment. It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

### Polynucleotides Having Promoter Activity

Native *C. glutamicum* promoters were identified that satisfy both of the following criteria: 1) represent a ladder of constitutive promoters, i.e., a plurality of promoters with incrementally increasing levels of promoter activity; and 2) encoded by short DNA sequences, ideally less than 100 base pairs. A published data set describing global gene expression levels in *C. glutamicum* ATCC 13032 (Lee et al., Biotechnol Lett (2013) 35:709-717) was examined to identify genes that were constitutively expressed across different growth conditions. Genes whose expression level remained constant (defined as a ratio of expression between 0.33 and 3) across two growth conditions, namely chemostat growth in minimal media with and without the addition of hydrogen peroxide satisfied the first criterion. A published data set describing the *C. glutamicum* ATCC 13032 transcriptome (Pfeifer-Sancar et al., BMC Genomics 2013, 14:888) was examined to find genes with compact promoters, *i.e.* those consisting of the 60 base pair core promoter region and a 5' untranslated region between 26 and 40 base pairs in length. The two data sets were cross-referenced to identify promoters that satisfied both criteria. See Figure 1. The following five wild-type promoters were identified (Table 1).

**Table 1: Promoters of C glutamicum Having Increasing Levels of Expression and Constituent Expression Under Different Growth Conditions**

| **Strain** | **SEQ ID NO** | **Mean Activity** |
|---|---|---|
| Pcg1864-eyfp | 2 | 89243 |
| Pcg0007-eyfp | 3 | 44527 |
| Pcg0755-eyfp | 4 | 43592 |
| Pcg3381-eyfp | 6 | 4723 |
| Pcg3121-eyfp | 8 | 98 |

The wild-type promoters cg1860, and cg3121 are not described in the literature. The wild-type promoter cg0007-*gyrB* is also not described in the literature, however, Neumann and Quiñones, (J Basic Microbiol. 1997;37(1):53-69) describes regulation of gyrB gene expression in *E. coli.* The wild-type promoter cg0755 is a known part of the methionine biosynthesis pathway (Suda et al., Appl Microbiol Biotechnol (2008) 81:505-513; and Rey et al., Journal of Biotechnology 103 (2003) 51-65). The wild-type promoter cg3381 is a *tatA* homolog. The tatA pathway in *Corynebacterium* is described by Kikuchi et al., Applied and Environmental Microbiology, Nov. 2006, p. 7183-7192. The strong constitutive promoter Pcg0007 was chosen for mutagenesis. Four out of six positions in the predicted -10 element (TAAGAT) of Pcg0007 were randomized to generate both stronger and attenuated promoter variants (SEQ ID NOs 1, 5, and 7),

Accordingly, one embodiment of the present invention relates to native promoters comprising polynucleotides isolated from *C. glutamicum,* and mutant promoters derived therefrom that together represent a ladder of constitutive promoters with incrementally increasing levels of promoter activity. In some embodiments a *C. glutamicum* promoter can be encoded by a short DNA sequence. In some embodiments a *C. glutamicum* promoter can be encoded by a DNA sequence of less than 100 base pairs.

One embodiment of the present invention relates to a promoter polynucleotide comprising a sequence selected from: SEQ ID NO:1 (Pcg0007_lib_39), SEQ ID NO:2 (Pcg1860), SEQ ID NO:3 (Pcg0007), SEQ ID NO:4 (Pcg0755), SEQ ID NO:5 (Pcg0007_lib_265), SEQ ID NO:6 (Pcg3381), SEQ ID NO:7 (Pcg0007_lib_119), or SEQ ID NO:8 (Pcg3121). In another embodiment, the present specification provides for, and includes, a promoter polynucleotide comprising of SEQ ID NO: 1 functionally linked to at least one heterologous target gene. In an embodiment, the present specification provides for, and includes, a promoter polynucleotide of SEQ ID NO:2 functionally linked to at least one heterologous target gene. In another embodiment, the present specification provides for, and includes, a promoter polynucleotide of SEQ ID NO:3 functionally linked to at least one heterologous target gene. In another embodiment, the present specification provides for, and includes, a promoter polynucleotide of SEQ ID NO:4 functionally linked to at least one heterologous target gene. In another embodiment, the present specification provides for, and includes, a promoter polynucleotide of SEQ ID NO:5 functionally linked to at least one heterologous target gene. In another embodiment, the present specification provides for, and includes, a promoter polynucleotide comprising of SEQ ID NO:5 functionally linked to at least one heterologous target gene. In another embodiment, the present specification provides for, and includes, a promoter polynucleotide of SEQ ID NO:7 functionally linked to at least one heterologous target gene. In another embodiment, the present specification provides for, and includes, a promoter polynucleotide of SEQ ID NO:8 functionally linked to at least one heterologous target gene.

As used herein, a "promoter cassette" refers to the polynucleotide sequences comprising a promoter polynucleotide of SEQ ID NOs:1 to 8 functionally linked to at least one heterologous target gene. In certain embodiments of the present disclosure, a "promoter cassette" may further include one or more of a linker polynucleotide, a transcription terminator following the heterologous gene, a ribosome binding site upstream of the start codon of the heterologous gene, and combinations of each. One embodiment of the present invention relates to a promoter polynucleotide consisting of a sequence selected from: SEQ ID NO:1, SEQ ID NO:2, SEQ ID NO:3, SEQ ID NO:4, SEQ ID NO:5, SEQ ID NO:6, SEQ ID NO:7, or SEQ ID NO:8. In an embodiment, the present specification provides for, and includes a promoter polynucleotide sequence of SEQ ID NO: 1. In an embodiment, the present specification provides for, and includes a promoter polynucleotide sequence of SEQ ID NO:5. In an embodiment, the present specification provides for, and includes a promoter polynucleotide sequence of SEQ ID NO:7. As used hereing a promoter cassette may described by reference the promoter name followed by the name of the heterologous target gene that is functionally linked to it. For example, the promoter of SEQ ID NO: 1, entitled Pcg1860, functionally linke to the gene zwf encoding the glucose-6-phosphate 1-dehydrogenase gene is referenced as Pcg1860-zwf. Similarly, Pcg0007_39-lysA is the 0007_39 promoter of SEQ ID NO:1 functionally linked to target gene lysA encoding the polypeptide diaminopimelate decarboxylase.

One embodiment of the present invention relates to combinations of the promoter polynucleotides described herein. In this context the term "combinations of promoter polynucleotides" refers to two or more polynucleotides that may be present as separate isolated sequences, as components of separate polynucleotide molecules, or as components of the same polynucleotide molecule, and combinations thereof. Examples of polynucleotide molecules include chromosomes and plasmids.

The invention also relates to an isolated promoter polynucleotide, which essentially consists of a polynucleotide having the nucleotide sequence depicted in SEQ ID NO:1, SEQ ID NO:2, SEQ ID NO:3, SEQ ID NO:4, SEQ ID NO:5, SEQ ID NO:6, SEQ ID NO:7, or SEQ ID NO:8. In an embodiment, the present specification provides for, and includes an isolated promoter polynucleotide of SEQ ID NO:1. In an embodiment, the present specification provides for, and includes an isolated promoter polynucleotide of SEQ ID NO:5. In an embodiment, the present specification provides for, and includes an isolated promoter polynucleotide of SEQ ID NO:7.

The term "essentially" in this context means that a polynucleotide of no more than 1,000, no more than 800, no more than 700, no more than 600, no more than 500 or no more than 400 nucleotides in length; and a polynucleotide of no more than 15,000, no more than 10,000, no more than 7,500, no more than 5,000, no more than 2,500, no more than 1,000, no more than 800, no more than 700, no more than 600, no more than 500, or no more than 400 nucleotides in length have been added to the 5' end and 3' end, respectively, of the polynucleotides of SEQ ID NO:1, SEQ ID NO:2, SEQ ID NO:3, SEQ ID NO:4, SEQ ID NO:5, SEQ ID NO:6, SEQ ID NO:7, or SEQ ID NO:8.

Any useful combination of the features from the preceding two lists of polynucleotides added to the 5' end and 3' end, respectively, of the polynucleotides of SEQ ID NO:1, SEQ ID NO:2, SEQ ID NO:3, SEQ ID NO:4, SEQ ID NO:5, SEQ ID NO:6, SEQ ID NO:7, or SEQ ID NO:8, is in accordance with the invention here. "Useful combination" means, for example, a combination of features which results in an efficient recombination being carried out. The use of additions of the same length flanking a DNA region to be replaced facilitates the transfer of the region by homologous recombination in the experimental procedure. Relatively long flanking homologous regions are advantageous for efficient recombination between circular DNA molecules but cloning of the replacement vector is made more difficult with increasing length of the flanks (Wang et al., Molecular Biotechnology, 432:43-53 (2006)). The specification provides for, and includes, homologous regions flanking a promoter polynucleotide sequence of SEQ ID NOs:1 to 8 functionally linked to at least on heterologous target gene (e.g., the "promoter cassette") to direct homologous recombination and replacement of a target gene sequence. In an embodiment, the homologus regions are direct repeat regions. In an embodiment, the homologous regions comprises between 500 base pairs (bp) and 5000 bp each of the target gene sequence flanking the promoter cassette. In an embodiment, the homologous regions comprises at least 500 bp each of the target gene sequence flanking the promoter cassette. In an embodiment, the homologous regions comprises at least 1000 bp (1 Kb) each of the target gene sequence flanking the promoter cassette. In an embodiment, the homologous regions comprises at least 2 Kb each of the target gene sequence flanking the promoter cassette. In an embodiment, the homologous regions comprises at least 5 Kb each of the target gene sequence flanking the promoter cassette.

The invention furthermore relates to an isolated promoter polynucleotide, which consists of the nucleotide sequence depicted in SEQ ID NO:1, SEQ ID NO:2, SEQ ID NO:3, SEQ ID NO:4, SEQ ID NO:5, SEQ ID NO:6, SEQ ID NO:7, or SEQ ID NO:8. In an embodiment, the isolate promoter polynucleotide consists of the polynucleotide sequence of SEQ ID NO:1. In an embodiment, the isolate promoter polynucleotide consists of the polynucleotide sequence of SEQ ID NO:5. In an embodiment, the isolate promoter polynucleotide consists of the polynucleotide sequence of SEQ ID NO:7.

Details regarding the biochemistry and chemical structure of polynucleotides as present in living things such as microorganisms, for example, can be found *inter alia* in the text book "Biochemie" [Biochemistry] by Berg et al. (Spektrum Akademischer Verlag Heidelberg Berlin, Germany, 2003; ISBN 3-8274-1303-6).

Polynucleotides consisting of deoxyribonucleotide monomers containing the nucleobases or bases adenine (A), guanine (G), cytosine (C) and thymine (T) are referred to as deoxyribo-polynucleotides or deoxyribonucleic acid (DNA). Polynucleotides consisting of ribonucleotide monomers containing the nucleobases or bases adenine (A), guanine (G), cytosine (C) and uracil (U) are referred to as ribopolynucleotides or ribonucleic acid (RNA). The monomers in said polynucleotides are covalently linked to one another by a 3',5'-phosphodiester bond.

A "promoter polynucleotide" or a "promoter" or a "polynucleotide having promoter activity" means a polynucleotide, preferably deoxyribopolynucleotide, or a nucleic acid, preferably deoxyribonucleic acid (DNA), which when functionally linked to a polynucleotide to be transcribed determines the point and frequency of initiation of transcription of the coding polynucleotide, thereby enabling the strength of expression of the controlled polynucleotide to be influenced. The term "promoter ladder" as used herein refers to a plurality of promoters with incrementally increasing levels of promoter activity. The term "promoter activity" as used herein refers to the ability of the promoter to initiate transcription of an polynucleotide sequence into mRNA. Methods of assessing promoter activity are well known to those of skill in the art and include, for example the methods described in Example 2 below. The term "constitutive promoter" as used herein refers to a promoter that directs the transcription of tits associated genes at a constant rate regardless of the internal or external cellular conditions.

Owing to the double-stranded structure of DNA, the strand complementary to the strand in SEQ ID NO:1, SEQ ID NO:2, SEQ ID NO:3, SEQ ID NO:4, SEQ ID NO:5, SEQ ID NO:6, SEQ ID NO:7, or SEQ ID NO:8 of the sequence listing is likewise a subject of the invention.

### Target Genes

One embodiment of the present invention relates to methods of expressing a target gene, comprising culturing a host cell transformed with a recombinant vector comprising a promoter polynucleotide as described herein. Target genes are polynucleotides the expression of which are controlled by the promoters described herein. The target genes may be coding polynucleotides which code for one or more polypeptide(s) or non-coding polynucleotides such as non-coding RNAs. A polynucleotide coding for a protein/polypeptide essentially consists of a start codon selected from the group consisting of ATG, GTG and TTG, preferably ATG or GTG, particularly preferably ATG, a protein-encoding sequence and one or more stop codon(s) selected from the group consisting of TAA, TAG and TGA.

"Transcription" means the process by which a complementary RNA molecule is produced starting from a DNA template. This process involves proteins such as RNA polymerase, "sigma factors" and transcriptional regulatory proteins. Where the target gene is a coding polynucleotide, the synthesized RNA (messenger RNA, mRNA) then serves as a template in the process of translation which subsequently yields the polypeptide or protein.

"Functionally linked" means in this context the sequential arrangement of the promoter polynucleotide according to the invention with a further oligo- or polynucleotide, resulting in transcription of said further polynucleotide to produce a sense RNA transcript.

If the further polynucleotide is a target gene which codes for a polypeptide/protein and consists of the coding region for a polypeptide, starting with a start codon, including the stop codon and, where appropriate, including a transcription termination sequence, "functionally linked" then means the sequential arrangement of the promoter polynucleotide according to the invention with the target gene, resulting in transcription of said target gene and translation of the synthesized RNA.

If the target gene codes for a plurality of proteins/polypeptides, each gene may be preceded by a ribosome-binding site. Where appropriate, a termination sequence is located downstream of the last gene.

The target gene preferably codes for one or more polypeptides or proteins of the biosynthetic pathway of biomolecules, preferably selected from the group of proteinogenic amino acids, non-proteinogenic amino acids, vitamins, nucleosides, nucleotides and organic acids. The target gene preferably consists of one or more of the polynucleotides listed in Table 1 of EP 1 108 790 A2.

The present specification provides for, and includes, recombinant nucleic acid molecules comprising a promoter polynucleotide sequence selected from the group consisting of SEQ ID NOs:1 to 8 functionally linked to any one of the heterologous target genes identifiable in the Kyoto Encyclopedia of Genes and Genomes (KEGG) as genes involved in metabolic and biosynthetic pathways. The KEGG database is available on the internet at genome.jp/kegg. In an embodiment, the promoter polynucleotide sequences of SEQ ID NOs:1 to 8 are functionally linked to one or more target genes of the lysine biosynthesis pathway as represented in KEGG map number 00300. In an embodiment, the one or more target genes are selected from the Lysine succinyl-DAP biosynthesis pathway, M00016. In an embodiment, the one or more target genes are selected from the lysine acetyl-DAP biosynthesis pathway, M00525. In an embodiment, the one or more target genes are selected from the lysine DAP dehydrogenase biosynthesis pathway, M00526. In an embodiment, the one or more target genes are selected from the lysine DAP aminotransferase biosynthesis pathway, M00527. In an embodiment, the one or more target genes are selected from the AAA pathway biosynthesis pathway, M00030. In an embodiment, the one or more target genes are selected from the lysine biosynthesis pathway from 2-oxoglutarate, M00433 or the lysine biosynthesis pathway mediated by LysW, M00031.

The present disclosure provides for, and includes, the promoter polynucleotide sequences of SEQ ID NOs:1 to 8 are functionally linked to one or more target genes of the the serine biosynthesis pathway comprising genes of entry M00020. In an embodiment, the promoter polynucleotide sequences of SEQ ID NOs:1 to 8 are functionally linked to one or more target genes of the threonine biosynthesis pathway comprising genes of KEGG entry M00018. In an embodiment, the promoter polynucleotide sequences of SEQ ID NOs:1 to 8 are functionally linked to one or more target genes of the cysteine biosynthesis pathway comprising genes of KEGG entry M00021. In an embodiment, the promoter polynucleotide sequences of SEQ ID NOs:1 to 8 are functionally linked to one or more target genes of the cysteine biosynthesis pathway comprising genes of KEGG entry M00338. In an embodiment, the promoter polynucleotide sequences of SEQ ID NOs:1 to 8 are functionally linked to one or more target genes of the cysteine biosynthesis pathway comprising genes of KEGG entry M00609. In an embodiment, the promoter polynucleotide sequences of SEQ ID NOs:1 to 8 are functionally linked to one or more target genes of the methionine biosynthesis pathway comprising genes of KEGG entry M00017. In an embodiment, the promoter polynucleotide sequences of SEQ ID NOs:1 to 8 are functionally linked to one or more target genes of the valine/isoleucine biosynthesis pathway comprising genes of KEGG entry M00019. In an embodiment, the promoter polynucleotide sequences of SEQ ID NOs:1 to 8 are functionally linked to one or more target genes of the isoleucine biosynthesis pathway comprising genes of KEGG entry M00535. In an embodiment, the promoter polynucleotide sequences of SEQ ID NOs:1 to 8 are functionally linked to one or more target genes of the isoleucine biosynthesis pathway comprising genes of KEGG entry M00570. In an embodiment, the promoter polynucleotide sequences of SEQ ID NOs:1 to 8 are functionally linked to one or more target genes of the leucine biosynthesis pathway comprising genes of KEGG entry M00432. In an embodiment, the promoter polynucleotide sequences of SEQ ID NOs:1 to 8 are functionally linked to one or more target genes of the proline biosynthesis pathway comprising genes of KEGG entry M00015. In an embodiment, the promoter polynucleotide sequences of SEQ ID NOs:1 to 8 are functionally linked to one or more target genes of the ornithine biosynthesis pathway comprising genes of KEGG entry M00028. In an embodiment, the promoter polynucleotide sequences of SEQ ID NOs:1 to 8 are functionally linked to one or more target genes of the ornithine biosynthesis pathway comprising genes of KEGG entry M00763. In an embodiment, the promoter polynucleotide sequences of SEQ ID NOs:1 to 8 are functionally linked to one or more target genes of the histidine biosynthesis pathway comprising genes of KEGG entry M00026. In an embodiment, the promoter polynucleotide sequences of SEQ ID NOs:1 to 8 are functionally linked to one or more target genes of the shikimate biosynthesis pathway comprising genes of KEGG entry M00022. In an embodiment, the promoter polynucleotide sequences of SEQ ID NOs:1 to 8 are functionally linked to one or more target genes of the tryptophan biosynthesis pathway comprising genes of entry M00023. In an embodiment, the promoter polynucleotide sequences of SEQ ID NOs:1 to 8 are functionally linked to one or more target genes of the phenylalanine biosynthesis pathway comprising genes of KEGG entry M00024. In an embodiment, the promoter polynucleotide sequences of SEQ ID NOs:1 to 8 are functionally linked to one or more target genes of the tyrosine biosynthesis pathway comprising genes of KEGG entry M00025. In an embodiment, the promoter polynucleotide sequences of SEQ ID NOs:1 to 8 are functionally linked to one or more target genes of the tyrosine biosynthesis pathway comprising genes of KEGG entry M00040.

The present disclosure provides for, and includes, the promoter polynucleotide sequences of SEQ ID NOs:1, 5 or 7 are functionally linked to one or more target genes of the the serine biosynthesis pathway comprising genes of entry M00020. In an embodiment, the promoter polynucleotide sequences of SEQ ID NOs:1, 5 or 7 are functionally linked to one or more target genes of the threonine biosynthesis pathway comprising genes of KEGG entry M00018. In an embodiment, the promoter polynucleotide sequences of SEQ ID NOs:1, 5 or 7 are functionally linked to one or more target genes of the cysteine biosynthesis pathway comprising genes of KEGG entry M00021. In an embodiment, the promoter polynucleotide sequences of SEQ ID NOs:1, 5 or 7 are functionally linked to one or more target genes of the cysteine biosynthesis pathway comprising genes of KEGG entry M00338. In an embodiment, the promoter polynucleotide sequences of SEQ ID NOs:1, 5 or 7 are functionally linked to one or more target genes of the cysteine biosynthesis pathway comprising genes of KEGG entry M00609. In an embodiment, the promoter polynucleotide sequences of SEQ ID NOs:1, 5 or 7 are functionally linked to one or more target genes of the methionine biosynthesis pathway comprising genes of KEGG entry M00017. In an embodiment, the promoter polynucleotide sequences of SEQ ID NOs:1, 5 or 7 are functionally linked to one or more target genes of the valine/isoleucine biosynthesis pathway comprising genes of KEGG entry M00019. In an embodiment, the promoter polynucleotide sequences of SEQ ID NOs:1, 5 or 7 are functionally linked to one or more target genes of the isoleucine biosynthesis pathway comprising genes of KEGG entry M00535. In an embodiment, the promoter polynucleotide sequences of SEQ ID NOs:1, 5 or 7 are functionally linked to one or more target genes of the isoleucine biosynthesis pathway comprising genes of KEGG entry M00570. In an embodiment, the promoter polynucleotide sequences of SEQ ID NOs:1, 5 or 7 are functionally linked to one or more target genes of the leucine biosynthesis pathway comprising genes of KEGG entry M00432. In an embodiment, the promoter polynucleotide sequences of SEQ ID NOs:1, 5 or 7 are functionally linked to one or more target genes of the proline biosynthesis pathway comprising genes of KEGG entry M00015. In an embodiment, the promoter polynucleotide sequences of SEQ ID NOs:1, 5 or 7 are functionally linked to one or more target genes of the ornithine biosynthesis pathway comprising genes of KEGG entry M00028. In an embodiment, the promoter polynucleotide sequences of SEQ ID NOs:1, 5 or 7 are functionally linked to one or more target genes of the ornithine biosynthesis pathway comprising genes of KEGG entry M00763. In an embodiment, the promoter polynucleotide sequences of SEQ ID NOs:1, 5 or 7 are functionally linked to one or more target genes of the histidine biosynthesis pathway comprising genes of KEGG entry M00026. In an embodiment, the promoter polynucleotide sequences of SEQ ID NOs:1, 5 or 7 are functionally linked to one or more target genes of the shikimate biosynthesis pathway comprising genes of KEGG entry M00022. In an embodiment, the promoter polynucleotide sequences of SEQ ID NOs:1, 5 or 7 are functionally linked to one or more target genes of the tryptophan biosynthesis pathway comprising genes of entry M00023. In an embodiment, the promoter polynucleotide sequences of SEQ ID NOs:1, 5 or 7 are functionally linked to one or more target genes of the phenylalanine biosynthesis pathway comprising genes of KEGG entry M00024. In an embodiment, the promoter polynucleotide sequences of SEQ ID NOs:1, 5 or 7 are functionally linked to one or more target genes of the tyrosine biosynthesis pathway comprising genes of KEGG entry M00025. In an embodiment, the promoter polynucleotide sequences of SEQ ID NOs:1, 5 or 7 are functionally linked to one or more target genes of the tyrosine biosynthesis pathway comprising genes of KEGG entry M00040.

The present specification provides for, and includes, recombinant nucleic acid molecules comprising a promoter polynucleotide sequence selected from the group consisting of SEQ ID NOs:1 to 8 functionally linked to any one of the heterologous target genes from *Corynebacterium glutamicum* ATCC 13032 provided in Table 2 or any *Corynebacterium glutamicum* equivalent thereof. Sequence start and end positions correspond to genomic nucleotide accession NC_003450.3. It will be understood by those of ordinary skill in the art that corresponding genes exist in other strains of *C. glutamicum* and may be readily identified from Table 2. In an embodiment, the present specification provides for, and includes a recombinant nucleic acid molecule comprising a promoter polynucleotide sequence of SEQ ID NO: 1 functionally linked to a heterologous target gene recited in Table 2. In an embodiment, the present specification provides for, and includes a recombinant nucleic acid molecule comprising a promoter polynucleotide sequence of SEQ ID NO:2 functionally linked to a heterologous target gene recited in Table 2. In an embodiment, the present specification provides for, and includes a recombinant nucleic acid molecule comprising a promoter polynucleotide sequence of SEQ ID NO:3 functionally linked to a heterologous target gene recited in Table 2. In an embodiment, the present specification provides for, and includes a recombinant nucleic acid molecule comprising a promoter polynucleotide sequence of SEQ ID NO:4 functionally linked to a heterologous target gene recited in Table 2. In an embodiment, the present specification provides for, and includes a recombinant nucleic acid molecule comprising a promoter polynucleotide sequence of SEQ ID NO:5 functionally linked to a heterologous target gene recited in Table 2. In an embodiment, the present specification provides for, and includes a recombinant nucleic acid molecule comprising a promoter polynucleotide sequence of SEQ ID NO:6 functionally linked to a heterologous target gene recited in Table 2. In an embodiment, the present specification provides for, and includes a recombinant nucleic acid molecule comprising a promoter polynucleotide sequence of SEQ ID NO:7 functionally linked to a heterologous target gene recited in Table 2. In an embodiment, the present specification provides for, and includes a recombinant nucleic acid molecule comprising a promoter polynucleotide sequence of SEQ ID NO:8 functionally linked to a heterologous target gene recited in Table 2.

**Table 2: Target genes from Corynebacterium glutamicum according to the present specification**

| Gene ID | Symbol | Aliases | description | start | end | orient ation |
|---|---|---|---|---|---|---|
| 1021315 | NCgl0248 | NCgl0248, Cgl0252 | aspartate-semialdehyde dehydrogenase | 270660 | 271694 | plus |
| 1021300 | NCgl0223 | NCgl0223, Cgl0226 | prephenate dehydrogenase | 241880 | 242902 | minus |
| 1021294 | NCgl0247 | NCgl0247, Cgl0251 | aspartate kinase | 269371 | 270636 | plus |
| 1021282 | NCgl0215 | NCgl0215, Cgl0218 | aminotransferase | 232257 | 233282 | minus |
| 1021250 | NCgl0181 | NCgl0181, Cgl0184 | glutamine 2-oxoglutarate aminotransferase large subunit | 195240 | 199772 | plus |
| 1021247 | gltD | NCgl0182, Cgl0185 | glutamate synthase | 199772 | 201292 | plus |
| 1021203 | aroE | NCgl0409, Cgl0424 | quinate/shikimate dehydrogenase | 446538 | 447389 | plus |
| 1021149 | NCgl0245 | NCgl0245, Cgl0248 | 2-isopropylmalate synthase | 266151 | 267896 | minus |
| 1021136 | gpmA | NCgl0390, Cgl0402 | 2,3-bisphosphoglycerate-dependent phosphoglycerate mutase | 425177 | 425923 | plus |
| 1021131 | NCgl0408 | NCgl0408, Cgl0423 | 3-dehydroquinate dehydratase | 446087 | 446524 | plus |
| 1021078 | NCgl0398 | NCgl0398, Cgl0410 | pyrroline-5-carboxylate reductase | 434877 | 435698 | plus |
| 1020978 | trpA | NCgl2932, Cgl3035 | tryptophan synthase subunit alpha | 3239333 | 3240175 | plus |
| 1020976 | NCgl2931 | NCgl2931, Cgl3034 | tryptophan synthase subunit beta | 3238083 | 3239336 | plus |
| 1020975 | NCgl2930 | NCgl2930, trpC, trpF | bifunctional indole-3-glycerol phosphate synthase/phosphoribosylant hranilate isomerase | 3236642 | 3238066 | plus |
| 1020974 | trpD | NCgl2929, Cgl3032 | anthranilate phosphoribosy ltransferase | 3235603 | 3236649 | plus |
| 1020973 | NCgl2928 | NCgl2928, Cgl3031 | anthranilate synthase II | 3234957 | 3235583 | plus |
| 1020972 | NCgl2927 | NCgl2927, Cgl3029 | anthranilate synthase I | 3233404 | 3234960 | plus |
| 1020852 | NCgl2809 | NCgl2809, Cgl2910 | pyruvate kinase | 3110462 | 3112321 | minus |
| 1020842 | NCgl2799 | NCgl2799, Cgl2899 | prephenate dehydratase | 3098576 | 3099523 | minus |
| 1020841 | NCgl2798 | NCgl2798, Cgl2898 | phosphoglycerate mutase | 3097902 | 3098573 | minus |
| 1020788 | NCgl2747 | NCgl2747, Cgl2844 | aminotransferase | 3030670 | 3031983 | plus |
| 1020745 | NCgl2704 | NCgl2704, Cgl2802 | nucleosidase | 2988212 | 2988772 | minus |
| 1020729 | NCgl2688 | NCgl2688, Cgl2786 | cystathionine gamma-synthase | 2972058 | 2973206 | minus |
| 1020714 | NCgl2673 | NCgl2673, Cgl2770 | fructose-bisphosphate aldolase | 2954239 | 2955273 | minus |
| 1020594 | NCgl2557 | NCgl2557, Cgl2646 | dihydrodipicolinate synthase | 2815459 | 2816397 | plus |
| 1020564 | NCgl2528 | NCgl2528, Cgl2617 | D-2-hydroxyisocaproate dehydrogenase | 2786754 | 2787716 | minus |
| 1020509 | NCgl2474 | NCgl2474, Cgl2563 | serine acetyltransferase | 2723065 | 2723613 | plus |
| 1020508 | NCgl2473 | NCgl2473, Cgl2562 | cysteine synthase | 2721905 | 2722861 | plus |
| 1020471 | NCgl2436 | NCgl2436, Cgl2522 | phosphoserine phosphatase | 2669555 | 2670856 | minus |
| 1020393 | NCgl2360 | NCgl2360, Cgl2446 | cystathionine gamma-synthase | 2590310 | 2591470 | minus |
| 1020370 | NCgl2337 | NCgl2337, Cgl2423 | ribose-5-phosphate isomerase B | 2563930 | 2564403 | minus |
| 1020307 | NCgl2274 | NCgl2274, Cgl2356 | gamma-glutamyl kinase | 2496668 | 2497777 | minus |
| 1020305 | proA | NCgl2272, Cgl2354 | gamma-glutamyl phosphate reductase | 2494337 | 2495635 | minus |
| 1020301 | NCgl2268 | NCgl2268, Cgl2350 | fructose-2,6-bisphosphatase | 2491149 | 2491859 | minus |
| 1020260 | NCgl2227 | NCgl2227, Cgl2309 | PLP-dependent aminotransferase | 2444607 | 2445713 | plus |
| 1020188 | NCgl2155 | NCgl2155, Cgl2236 | bifunctional RNase H/acid phosphatase | 2371410 | 2372558 | minus |
| 1020181 | NCgl2148 | NCgl2148, Cgl2229 | glutamine synthase | 2362816 | 2364156 | minus |
| 1020172 | NCgl2139 | NCgl2139, Cgl2220 | threonine synthase | 2353598 | 2355043 | minus |
| 1020166 | NCgl2133 | NCgl2133, Cgl2214 | glutamine synthase | 2348830 | 2350263 | plus |
| 1020155 | NCgl2123 | NCgl2123, Cgl2204 | branched-chain amino acid aminotransferase | 2335913 | 2337016 | minus |
| 1020130 | NCgl2098 | NCgl2098, Cgl2178 | 3-deoxy-7-phosphoheptulonate synthase | 2307695 | 2309095 | minus |
| 1020087 | NCgl2055 | NCgl2055, Cgl2136 | cysteine synthase | 2258360 | 2259313 | minus |
| 1020086 | NCgl2054 | NCgl2054, Cgl2135 | diaminopimelate decarboxylase | 2255736 | 2257025 | minus |
| 1020080 | NCgl2048 | NCgl2048, Cgl2129 | methionine synthase II | 2247004 | 2248209 | minus |
| 1020078 | NCgl2046 | NCgl2046, Cgl2127 | threonine dehydratase | 2244862 | 2246172 | minus |
| 1020053 | hisD | NCgl2021, Cgl2102 | histidinol dehydrogenase | 2217597 | 2218925 | minus |
| 1020052 | NCgl2020 | NCgl2020, Cgl2101 | histidinol-phosphate aminotransferase | 2216491 | 2217591 | minus |
| 1020051 | hisB | NCgl2019, Cgl2100 | imidazoleglycerol-phosphate dehydratase | 2215866 | 2216474 | minus |
| 1020048 | hisH | NCgl2016, Cgl2097 | imidazole glycerol phosphate synthase subunit HisH | 2212638 | 2213273 | minus |
| 1020047 | NCgl2015 | NCgl2015, Cgl2096 | phosphoribosyl isomerase A | 2211879 | 2212619 | minus |
| 1020045 | hisF | NCgl2013, Cgl2094 | imidazole glycerol phosphate synthase subunit HisF | 2210270 | 2211046 | minus |
| 1020044 | hisI | NCgl2012, Cgl2093 | phosphoribosyl-AMP cyclohydrolase | 2209917 | 2210273 | minus |
| 1020042 | NCgl2010 | NCgl2010, Cgl2091 | indole-3-glycerol phosphate synthase | 2208364 | 2209149 | minus |
| 1020040 | NCgl2008 | NCgl2008, Cgl2089 | pyruvate kinase | 2205665 | 2207092 | minus |
| 1019930 | NCgl1898 | NCgl1898, Cgl1973 | 4-hydroxy-tetrahydrodipicolinate reductase | 2081188 | 2081934 | minus |
| 1019928 | dapA | NCgl1896, Cgl1971 | 4-hydroxy-tetrahydrodipicolinate synthase | 2079278 | 2080183 | minus |
| 1019900 | dapF | NCgl1868, Cgl1943 | diaminopimelate epimerase | 2051842 | 2052675 | minus |
| 1019614 | NCgl1583 | NCgl1583, Cgl1645 | L-serine deaminase | 1744884 | 1746233 | plus |
| 1019598 | aroE | NCgl1567, Cgl1629 | shikimate 5-dehydrogenase | 1724609 | 1725439 | minus |
| 1019592 | NCgl1561 | NCgl1561, Cgl1623 | chorismate synthase | 1719666 | 1720898 | minus |
| 1019591 | aroK | NCgl1560, Cgl1622 | shikimate kinase | 1719104 | 1719676 | minus |
| 1019590 | aroB | NCgl1559, Cgl1621 | 3-dehydroquinate synthase | 1717935 | 1719032 | minus |
| 1019571 | NCgl1541 | NCgl1541, Cgl1603 | methionine adenosyltransferase | 1699174 | 1700397 | minus |
| 1019566 | NCgl1536 | NCgl1536, Cgl1598 | ribulose-phosphate 3-epimerase | 1693259 | 1693918 | minus |
| 1019556 | NCgl1526 | NCgl1526, Cgl1588 | glyceraldehyde-3 -phosphate dehydrogenase | 1682621 | 1683625 | minus |
| 1019555 | pgk | NCgl1525, Cgl1587 | phosphoglycerate kinase | 1681187 | 1682404 | minus |
| 1019554 | tpiA | NCgl1524, Cgl1586 | triosephosphate isomerase | 1680329 | 1681108 | minus |
| 1019550 | NCgl1520 | NCgl1520, Cgl1582 | ornithine cyclodeaminase | 1674120 | 1675268 | minus |
| 1019543 | NCgl1513 | NCgl1513, Cgl1575 | transaldolase | 1666673 | 1667755 | plus |
| 1019542 | NCgl1512 | NCgl1512, Cgl1574 | transketolase | 1664403 | 1666505 | plus |
| 1019512 | NCgl1482 | NCgl1482, Cgl1540 | aconitate hydratase | 1626279 | 1629110 | plus |
| 1019480 | NCgl1450 | NCgl1450, Cgl1507 | methionine synthase I cobalamin-binding subunit | 1587570 | 1591235 | minus |
| 1019478 | hisE | NCgl1448, Cgl1505 | phosphoribosyl- A TP pyrophosphatase | 1586462 | 1586725 | minus |
| 1019477 | hisG | NCgl1447, Cgl1504 | ATP phosphoribosy ltransferase | 1585600 | 1586445 | minus |
| 1019377 | NCgl1347 | NCgl1347, Cgl1401 | argininosuccinate lyase | 1471477 | 1472910 | plus |
| 1019376 | NCgl1346 | NCgl1346, Cgl1400 | argininosuccinate synthase | 1470211 | 1471416 | plus |
| 1019374 | NCgl1344 | NCgl1344, Cgl1398 | ornithine carbamoyltransferase | 1468565 | 1469524 | plus |
| 1019373 | argD | NCgl1343, Cgl1397 | acetylornithine aminotransferase | 1467376 | 1468551 | plus |
| 1019372 | NCgl1342 | NCgl1342, Cgl1396 | acetylglutamate kinase | 1466422 | 1467375 | plus |
| 1019371 | argJ | NCgl1341, Cgl1395 | bifunctional ornithine acetyltransferase/N-acetylglutamate synthase | 1465210 | 1466376 | plus |
| 1019370 | argC | NCgl1340, Cgl1394 | N-acetyl-gamma-glutamyl-phosphate reductase | 1464053 | 1465126 | plus |
| 1019293 | leuD | NCgl1263, Cgl1316 | 3-isopropylmalate dehydratase small subunit | 1381902 | 1382495 | plus |
| 1019292 | NCgl1262 | NCgl1262, Cgl1315 | 3-isopropylmalate dehydratase large subunit | 1380440 | 1381885 | plus |
| 1019267 | NCgl1237 | NCgl1237, Cgl1286 | 3-isopropylmalate dehydrogenase | 1353489 | 1354511 | plus |
| 1019265 | NCgl1235 | NCgl1235, Cgl1284 | D-3-phosphoglycerate dehydrogenase | 1350855 | 1352447 | plus |
| 1019254 | NCgl1224 | NCgl1224, Cgl1273 | ketol-acid reductoisomerase | 1340724 | 1341740 | plus |
| 1019253 | ilvH | NCgl1223, Cgl1272 | acetolactate synthase small subunit | 1340025 | 1340543 | plus |
| 1019252 | NCgl1222 | NCgl1222, Cgl1271 | acetolactate synthase large subunit | 1338131 | 1340011 | plus |
| 1019249 | NCgl1219 | NCgl1219, Cgl1268 | dihydroxy-acid dehydratase | 1333439 | 1335280 | minus |
| 1019232 | NCgl1202 | NCgl1202, Cgl1250 | 6-phosphofructokinase | 1315046 | 1316086 | plus |
| 1019167 | NCgl1137 | NCgl1137, Cgl1184 | homoserine kinase | 1243855 | 1244784 | plus |
| 1019166 | NCgl1136 | NCgl1136, Cgl1183 | homoserine dehydrogenase | 1242507 | 1243844 | plus |
| 1019163 | NCgl1133 | NCgl1133, Cgl1180 | diaminopimelate decarboxylase | 1239929 | 1241266 | plus |
| 1019124 | NCgl1094 | NCgl1094, Cgl1139 | 5-methyltetrahydropteroyltrigl utamate--homocysteine S-methyltransferase | 1188385 | 1190622 | minus |
| 1019117 | aroE | NCgl1087, Cgl1132 | shikimate 5-dehydrogenase | 1180869 | 1181675 | minus |
| 1019094 | NCgl1064 | NCgl1064, Cgl1109 | succinyl-diaminopimelate desuccinylase | 1155731 | 1156840 | plus |
| 1019093 | NCgl1063 | NCgl1063, Cgl1108 | tetrahydrodipicolinate N-succinyltransferase | 1154726 | 1155676 | minus |
| 1019091 | NCgl1061 | NCgl1061, Cgl1106 | 2,3,4,5 -tetrahydropyridine-2,6-dicarboxylate N-succinyltransferase | 1152370 | 1153263 | minus |
| 1019042 | NCgl1013 | NCgl1013, Cgl1058 | phosphoglycerate mutase | 1107503 | 1108204 | plus |
| 1018983 | glyA | NCgl0954, Cgl0996 | serine hydroxymethyltransferase | 1050624 | 1051928 | plus |
| 1018979 | NCgl0950 | NCgl0950, Cgl0990 | phospho-2-dehydro-3-deoxyheptonate aldolase | 1046610 | 1047710 | plus |
| 1018968 | NCgl0939 | NCgl0939, Cgl0978 | threonine dehydratase | 1038718 | 1039650 | minus |
| 1018964 | eno | NCgl0935, Cgl0974 | phosphopyruvate hydratase | 1034949 | 1036226 | plus |
| 1018934 | NCgl0905 | NCgl0905, Cgl0942 | ribose-phosphate pyrophosphokinase | 997463 | 998440 | minus |
| 1018929 | NCgl0900 | NCgl0900, Cgl0937 | glyceraldehyde-3 -phosphate dehydrogenase | 993174 | 994616 | plus |
| 1018848 | NCgl0819 | NCgl0819, Cgl0853 | hypothetical protein | 910852 | 911157 | minus |
| 1018824 | gltA | NCgl0795, Cgl0829 | type II citrate synthase | 877838 | 879151 | plus |
| 1018823 | NCgl0794 | NCgl0794, Cgl0828 | phosphoserine aminotransferase | 875982 | 877112 | minus |
| 1018809 | NCgl0780 | NCgl0780, Cgl0814 | aminotransferase | 861592 | 862755 | plus |
| 1018794 | NCgl0765 | NCgl0765, Cgl0799 | fructose-1,6-bisphosphatase | 841514 | 842296 | minus |
| 1018759 | NCgl0730 | NCgl0730, Cgl0764 | 3-phosphoshikimate 1-carboxyvinyltransferase | 801187 | 802479 | minus |
| 1018688 | NCgl0659 | NCgl0659, Cgl0689 | pyruvate carboxylase | 705211 | 708633 | plus |
| 1018663 | NCgl0634 | NCgl0634, Cgl0664 | monomeric isocitrate dehydrogenase (NADP+) | 677828 | 680044 | minus |

In an embodiment, the present specification provides for, and includes a recombinant vector comprising a promoter polynucleotide sequence of SEQ ID NO:1 functionally linked to a heterologous target gene recited in Table 2. In an embodiment, the present specification provides for, and includes a recombinant vector comprising a promoter polynucleotide sequence of SEQ ID NO:2 functionally linked to a heterologous target gene recited in Table 2. In an embodiment, the present specification provides for, and includes a recombinant vector comprising a promoter polynucleotide sequence of SEQ ID NO:3 functionally linked to a heterologous target gene recited in Table 2. In an embodiment, the present specification provides for, and includes a recombinant vector comprising a promoter polynucleotide sequence of SEQ ID NO:4 functionally linked to a heterologous target gene recited in Table 2. In an embodiment, the present specification provides for, and includes a recombinant vector comprising a promoter polynucleotide sequence of SEQ ID NO:5 functionally linked to a heterologous target gene recited in Table 2. In an embodiment, the present specification provides for, and includes a recombinant vector comprising a promoter polynucleotide sequence of SEQ ID NO:6 functionally linked to a heterologous target gene recited in Table 2. In an embodiment, the present specification provides for, and includes a recombinant vector comprising a promoter polynucleotide sequence of SEQ ID NO:7 functionally linked to a heterologous target gene recited in Table 2. In an embodiment, the present specification provides for, and includes a recombinant vector comprising a promoter polynucleotide sequence of SEQ ID NO:8 functionally linked to a heterologous target gene recited in Table 2.

In an embodiment, the present specification provides for, and includes, a host cell transformed with a recombinant vector comprising a promoter polynucleotide sequence of SEQ ID NO:1 functionally linked to a heterologous target gene recited in Table 2. In an embodiment, the present specification provides for, and includes, a host cell transformed with a recombinant vector comprising a promoter polynucleotide sequence of SEQ ID NO:2 functionally linked to a heterologous target gene recited in Table 2. In an embodiment, the present specification provides for, and includes, a host cell transformed with a recombinant vector comprising a promoter polynucleotide sequence of SEQ ID NO:3 functionally linked to a heterologous target gene recited in Table 2. In an embodiment, the present specification provides for, and includes, a host cell transformed with a recombinant vector comprising a promoter polynucleotide sequence of SEQ ID NO:4 functionally linked to a heterologous target gene recited in Table 2. In an embodiment, the present specification provides for, and includes, a host cell transformed with a recombinant vector comprising a promoter polynucleotide sequence of SEQ ID NO:5 functionally linked to a heterologous target gene recited in Table 2. In an embodiment, the present specification provides for, and includes, a host cell transformed with a recombinant vector comprising a promoter polynucleotide sequence of SEQ ID NO:6 functionally linked to a heterologous target gene recited in Table 2. In an embodiment, the present specification provides for, and includes, a host cell transformed with a recombinant vector comprising a promoter polynucleotide sequence of SEQ ID NO:7 functionally linked to a heterologous target gene recited in Table 2. In an embodiment, the present specification provides for, and includes, a host cell transformed with a recombinant vector comprising a promoter polynucleotide sequence of SEQ ID NO:8 functionally linked to a heterologous target gene recited in Table 2. As used herein, a host cell refers to an organisms described below in the section entitled 'Expression' that have been transformed with one or more of the promoter cassettes. As will be apparent to one of ordinary skill in the art, a host cell may comprise one or more promoter cassettes as described herein.

In an embodiment, the present specification provides for, and includes, a host cell transformed with a recombinant vector comprising a promoter polynucleotide sequence of SEQ ID NO:1 functionally linked to a heterologous target gene recited in Table 3. In an embodiment, the present specification provides for, and includes, a host cell transformed with a recombinant vector comprising a promoter polynucleotide sequence of SEQ ID NO:2 functionally linked to a heterologous target gene recited in Table 3. In an embodiment, the present specification provides for, and includes, a host cell transformed with a recombinant vector comprising a promoter polynucleotide sequence of SEQ ID NO:3 functionally linked to a heterologous target gene recited in Table 3. In an embodiment, the present specification provides for, and includes, a host cell transformed with a recombinant vector comprising a promoter polynucleotide sequence of SEQ ID NO:4 functionally linked to a heterologous target gene recited in Table 3. In an embodiment, the present specification provides for, and includes, a host cell transformed with a recombinant vector comprising a promoter polynucleotide sequence of SEQ ID NO:5 functionally linked to a heterologous target gene recited in Table 3. In an embodiment, the present specification provides for, and includes, a host cell transformed with a recombinant vector comprising a promoter polynucleotide sequence of SEQ ID NO:6 functionally linked to a heterologous target gene recited in Table 3. In an embodiment, the present specification provides for, and includes, a host cell transformed with a recombinant vector comprising a promoter polynucleotide sequence of SEQ ID NO:7 functionally linked to a heterologous target gene recited in Table 3. In an embodiment, the present specification provides for, and includes, a host cell transformed with a recombinant vector comprising a promoter polynucleotide sequence of SEQ ID NO:8 functionally linked to a heterologous target gene recited in Table 3. As used herein, a host cell refers to an organisms described below in the section entitled 'Expression' that have been transformed with one or more of the promoter cassettes. As will be apparent to one of ordinary skill in the art, a host cell may comprise one or more promoter cassettes as described herein.

**Table 3: C. glutamican L-lysine Biosynthetic Pathway**

| Symbol | Gene Name (EC #) | *C*. *Glutamicum* Gene | Position | Expression |
|---|---|---|---|---|
| asd | aspartate-semialdehyde dehydrogenase (EC: 1.2.1.11) | asd | 270660..271694 | + |
| dapA | 4-hydroxy-tetrahydrodipicolinate synthase (EC:4.3.3.7) | dapA | Complement (2079278..2080183 ) | + |
| dapB | dihydrodipicolinate reductase (EC:1.17.1.8) | Cgl1973 | complement(20811 88..2081934) | + |
| dapD | 2,3,4,5-tetrahydropyridine-2-carboxylate N-succinyltransferase (EC:2.3.1.117) | dapD | complement(11538 38..1154731) | + |
| dapD | 2,3,4,5-tetrahydropyridine-2-carboxylate N-succinyltransferase (EC:2.3.1.117) | dapD2 | complement(11561 94..1157144) | |
| cg0931 | N-succinyldiaminopimelate aminotransferase (EC:2.6.1.17) | cg0931 | 863063..864226 | + |
| dapE | succinyl-diaminopimelate | dapE | 1157199..1158308 | + |
| | desuccinylase (EC: 3.5.1.18) | | | |
| dapF | diaminopimelate epimerase (EC:5.1.1.7) | dapF | complement(20218 91..2022724) | + |
| lysA | diaminopimelate decarboxylase (EC:4.1.1.20) | lysA | 1241397..1242734 | + |
| ddh | diaminopimelate dehydrogenase (EC: 1.4.1.16) | ddh | complement(27600 62..2761024) | + |
| ask (lysC) | Aspartokinase Lysc Alpha And Beta Subunits (EC:2.7.2.4) | lysC | 269371..270636 | + |
| aspB | Aspartate Aminotransferase (EC:2.6.1.1) | aspB | 256618..257898 | + |
| PTS | Phosphotransferase System (PTS); Glucose-Specific Enzyme II BC Component Of PTS (EC:2.7.1.69) | ptsG | 1424684..1426735 | + |
| zwf | glucose-6-phosphate 1-dehydrogenase (EC: 1.1.1.49 1.1.1.363) | zwf | 1669327..1670871 | + |
| pgi | glucose-6-phosphate isomerase (EC:5.3.1.9) | pgi | complement(90922 7..910849) | + |
| tkt | transketolase (EC:2.2.1.1) | tkt | 1665870..1667972 | + |
| fbp | 6-phosphofructokinase 1 (EC:2.7.1.11) | Cgl1250 | 1315046..1316086 | + |
| ppc | phosphoenolpyruvate carboxylase (EC:4.1.1.31) | ppc | complement(16788 51..1681610) | + |
| pyc | pyruvate carboxylase (EC:6.4.1.1) | pyc | 706684..710106 | + |
| icd | isocitrate dehydrogenase (EC: 1.1.1.42) | icd | complement(67930 1..681517) | - |
| pck | phosphoenolpyruvate carboxykinase (GTP) (EC:4.1.1.32) | pck | complement(30253 65..3027197) | - |
| odx | Oxaloacetate decarboxylase (EC 4.1.1.3) | odx | AP017369.1:15089 67..1509782 (from C. glutamicum N24) | - |
| hom | homoserine kinase (EC:2.7.1.39) | Cgl1184 | 1243855..1244784 | - |
| | homoserine dehydrogenase (EC: 1.1.1.3); | Cgl1183 | 1242507..1243844 | - |
| | threonine synthase (EC:4.2.3.1) | Cgl2220 | complement(23535 98..2355043) | - |

In an embodiment, the present specification provides for, and includes a recombinant vector comprising a promoter polynucleotide sequence of SEQ ID NO:1 functionally linked to a heterologous target gene recited in Table 3. In an embodiment, the present specification provides for, and includes a recombinant vector comprising a promoter polynucleotide sequence of SEQ ID NO:2 functionally linked to a heterologous target gene recited in Table 3. In an embodiment, the present specification provides for, and includes a recombinant vector comprising a promoter polynucleotide sequence of SEQ ID NO:3 functionally linked to a heterologous target gene recited in Table 3. In an embodiment, the present specification provides for, and includes a recombinant vector comprising a promoter polynucleotide sequence of SEQ ID NO:4 functionally linked to a heterologous target gene recited in Table 3. In an embodiment, the present specification provides for, and includes a recombinant vector comprising a promoter polynucleotide sequence of SEQ ID NO:5 functionally linked to a heterologous target gene recited in Table 3. In an embodiment, the present specification provides for, and includes a recombinant vector comprising a promoter polynucleotide sequence of SEQ ID NO:6 functionally linked to a heterologous target gene recited in Table 3. In an embodiment, the present specification provides for, and includes a recombinant vector comprising a promoter polynucleotide sequence of SEQ ID NO:7 functionally linked to a heterologous target gene recited in Table 3. In an embodiment, the present specification provides for, and includes a recombinant vector comprising a promoter polynucleotide sequence of SEQ ID NO:8 functionally linked to a heterologous target gene recited in Table 3.

In an embodiment, the present specification provides for, and includes, a host cell transformed with a recombinant vector comprising a promoter polynucleotide sequence of SEQ ID NO:1 functionally linked to a heterologous target gene recited in Table 3. In an embodiment, the present specification provides for, and includes, a host cell transformed with a recombinant vector comprising a promoter polynucleotide sequence of SEQ ID NO:2 functionally linked to a heterologous target gene recited in Table 3. In an embodiment, the present specification provides for, and includes, a host cell transformed with a recombinant vector comprising a promoter polynucleotide sequence of SEQ ID NO:3 functionally linked to a heterologous target gene recited in Table 3. In an embodiment, the present specification provides for, and includes, a host cell transformed with a recombinant vector comprising a promoter polynucleotide sequence of SEQ ID NO:4 functionally linked to a heterologous target gene recited in Table 3. In an embodiment, the present specification provides for, and includes, a host cell transformed with a recombinant vector comprising a promoter polynucleotide sequence of SEQ ID NO:5 functionally linked to a heterologous target gene recited in Table 3. In an embodiment, the present specification provides for, and includes, a host cell transformed with a recombinant vector comprising a promoter polynucleotide sequence of SEQ ID NO:6 functionally linked to a heterologous target gene recited in Table 3. In an embodiment, the present specification provides for, and includes, a host cell transformed with a recombinant vector comprising a promoter polynucleotide sequence of SEQ ID NO:7 functionally linked to a heterologous target gene recited in Table 3. In an embodiment, the present specification provides for, and includes, a host cell transformed with a recombinant vector comprising a promoter polynucleotide sequence of SEQ ID NO:8 functionally linked to a heterologous target gene recited in Table 3. As used herein, a host cell refers to an organisms described below in the section entitled 'Expression' that have been transformed with one or more of the promoter cassettes. As will be apparent to one of ordinary skill in the art, a host cell may comprise one or more promoter cassettes as described herein.

The present specification provides for a host cell transformed with a recombinant vector comprising a promoter polynucleotide sequence selected from the group consisting of SEQ ID NOs:1 to 8 functionally linked to any one of the heterologous target genes from *Corynebacterium glutamicum* ATCC 13032 provided in Table 4 or their *Corynebacterium glutamicum* equivalent therof. Sequence start and end positions correspond to genomic nucleotide accession NC_003450.3. It will be understood by those of ordinary skill in the art that corresponding genes exist in other strains of *C. glutamicum* and may be readily identified from Table 4. In an embodiment, the present specification provides for, and includes, a host cell transformed with a recombinant vector comprising a promoter polynucleotide sequence of SEQ ID NO:1 functionally linked to a heterologous target gene recited in Table 4. In an embodiment, the present specification provides for, and includes, a host cell transformed with a recombinant vector comprising a promoter polynucleotide sequence of SEQ ID NO:2 functionally linked to a heterologous target gene recited in Table 4. In an embodiment, the present specification provides for, and includes, a host cell transformed with a recombinant vector comprising a promoter polynucleotide sequence of SEQ ID NO:3 functionally linked to a heterologous target gene recited in Table 4. In an embodiment, the present specification provides for, and includes, a host cell transformed with a recombinant vector comprising a promoter polynucleotide sequence of SEQ ID NO:4 functionally linked to a heterologous target gene recited in Table 4. In an embodiment, the present specification provides for, and includes, a host cell transformed with a recombinant vector comprising a promoter polynucleotide sequence of SEQ ID NO:5 functionally linked to a heterologous target gene recited in Table 4. In an embodiment, the present specification provides for, and includes, a host cell transformed with a recombinant vector comprising a promoter polynucleotide sequence of SEQ ID NO:6 functionally linked to a heterologous target gene recited in Table 4. In an embodiment, the present specification provides for, and includes, a host cell transformed with a recombinant vector comprising a promoter polynucleotide sequence of SEQ ID NO:7 functionally linked to a heterologous target gene recited in Table 4. In an embodiment, the present specification provides for, and includes, a host cell transformed with a recombinant vector comprising a promoter polynucleotide sequence of SEQ ID NO:8 functionally linked to a heterologous target gene recited in Table 4. As used herein, a host cell refers to an organisms described below in the section entitled 'Expression' that have been transformed with one or more of the promoter cassettes. As will be apparent to one of ordinary skill in the art, a host cell may comprise one or more promoter cassettes as described herein.

**Table 4: C. glutamican L-methionine Biosynthetic Pathway**

| Symbol | Gene Name (EC #) | *C. Glutamicum* Gene | Position |
|---|---|---|---|
| lysC | aspartate kinase [EC:2.7.2.4] | Cgl0251 | 269371..270636 |
| | aspartate-semialdehyde dehydrogenase [EC: 1.2.1.11] | Cgl0252 | 270660..271694 |
| dapA | 4-hydroxy-tetrahydrodi picolinate synthase [EC:4.3.3.7] | dapA | complement(2079278.. 2080183) |
| dapA | 4-hydroxy-tetrahydrodi picolinate synthase [EC:4.3.3.7] | Cgl2646 | 2815459..2816397 |
| dapB | 4-hydroxy-tetrahydrodi picolinate reductase [EC: 1.17.1.8] | Cgl1973 | complement(2081188.. 2081934) |
| dapD | 2,3,4,5-tetrahydropyridine-2-carboxylate N-succinyltransferase [EC:2.3.1.117] | Cgl1106 | complement(1152370.. 1153263) |
| dapC | N-succinyldiaminopimelate aminotransferase [EC:2.6.1.17] | Cgl0814 | 861592..862755 |
| dapE | succinyl-diaminopimelate desuccinylase [EC:3.5.1.18] | Cgl1109 | 1155731..1156840 |
| dapF | diaminopimelate epimerase [EC: 5.1.1.7] | dapF | complement(2051842.. 2052675) |
| lysA | diaminopimelate decarboxylase [EC:4.1.1.20] | Cgl1180 | 1239929..1241266 |

In an embodiment, the present specification provides for, and includes a recombinant vector comprising a promoter polynucleotide sequence of SEQ ID NO:3 functionally linked to a heterologous target gene recited in Table 4. In an embodiment, the present specification provides for, and includes a recombinant vector comprising a promoter polynucleotide sequence of SEQ ID NO:4 functionally linked to a heterologous target gene recited in Table 4. In an embodiment, the present specification provides for, and includes a recombinant vector comprising a promoter polynucleotide sequence of SEQ ID NO:5 functionally linked to a heterologous target gene recited in Table 4. In an embodiment, the present specification provides for, and includes a recombinant vector comprising a promoter polynucleotide sequence of SEQ ID NO:6 functionally linked to a heterologous target gene recited in Table 4. In an embodiment, the present specification provides for, and includes a recombinant vector comprising a promoter polynucleotide sequence of SEQ ID NO:7 functionally linked to a heterologous target gene recited in Table 4. In an embodiment, the present specification provides for, and includes a recombinant vector comprising a promoter polynucleotide sequence of SEQ ID NO:8 functionally linked to a heterologous target gene recited in Table 4.

In an embodiment, the present specification provides for, and includes, a host cell transformed with a recombinant vector comprising a promoter polynucleotide sequence of SEQ ID NO:1 functionally linked to a heterologous target gene recited in Table 4. In an embodiment, the present specification provides for, and includes, a host cell transformed with a recombinant vector comprising a promoter polynucleotide sequence of SEQ ID NO:2 functionally linked to a heterologous target gene recited in Table 4. In an embodiment, the present specification provides for, and includes, a host cell transformed with a recombinant vector comprising a promoter polynucleotide sequence of SEQ ID NO:3 functionally linked to a heterologous target gene recited in Table 4. In an embodiment, the present specification provides for, and includes, a host cell transformed with a recombinant vector comprising a promoter polynucleotide sequence of SEQ ID NO:4 functionally linked to a heterologous target gene recited in Table 4. In an embodiment, the present specification provides for, and includes, a host cell transformed with a recombinant vector comprising a promoter polynucleotide sequence of SEQ ID NO:5 functionally linked to a heterologous target gene recited in Table 4. In an embodiment, the present specification provides for, and includes, a host cell transformed with a recombinant vector comprising a promoter polynucleotide sequence of SEQ ID NO:6 functionally linked to a heterologous target gene recited in Table 4. In an embodiment, the present specification provides for, and includes, a host cell transformed with a recombinant vector comprising a promoter polynucleotide sequence of SEQ ID NO:7 functionally linked to a heterologous target gene recited in Table 4. In an embodiment, the present specification provides for, and includes, a host cell transformed with a recombinant vector comprising a promoter polynucleotide sequence of SEQ ID NO:8 functionally linked to a heterologous target gene recited in Table 4.

In some embodiments the target gene is associated with a biosynthetic pathway producing a biomolecule selected from: amino acids, organic acids, flavors and fragrances, biofuels, proteins and enzymes, polymers/monomers and other biomaterials, lipids, nucleic acids, small molecule therapeutics, protein therapeutics, fine chemicals, and nutraceuticals.

In some embodiments the target gene is associated with a biosynthetic pathway producing a secondary metabolite selected from: antibiotics, alkaloids, terpenoids, and polyketides. In some embodiments the target gene is associated with a metabolic pathway producing a primary metabolite selected from: alcohols, amino acids, nucleotides, antioxidants, organic acids, polyols, vitamins, and lipids/fatty acids. In some embodiments the target gene is associated with a biosynthetic pathway producing a macromolecule selected from: proteins, nucleic acids, and polymers

In addition it may be advantageous for the production of L-amino acids to enhance, in particular to overexpress one or more enzymes of the respective biosynthesis pathway, glycolysis, anaplerosis, citric acid cycle, pentose phosphate cycle, amino acid export and optionally regulatory proteins.

Thus for example, for the production of L-amino acids, it may be advantageous for one or more genes selected from the following group to be enhanced, in particular overexpressed: the gene dapA coding for dihydrodipicolinate synthase (EP-B 0 197 335); the gene *eno* coding for enolase (DE: 19947791.4); the gene *gap* coding for glyceraldehyde-3-phosphate dehydrogenase (Eikmanns (1992), Journal of Bacteriology 174:6076-6086); the gene *tpi* coding for triosephosphate isomerase (Eikmanns (1992), Journal of Bacteriology 174:6076-6086); the gene *pgk* coding for 3-phosphoglycerate kinase (Eikmanns (1992), Journal of Bacteriology 174:6076-6086); the gene zwf coding for glucose-6-phosphate dehydrogenase (JP-A-09224661); the gene *pyc* coding for pyruvate carboxylase (DE-A-198 31 609; Eikmanns (1992), Journal of Bacteriology 174:6076-6086); the gene *mqo* coding for malate-quinone-oxidoreductase (Molenaar et al., European Journal of Biochemistry 254, 395-403 (1998)); the gene *lysC* coding for a feedback-resistant aspartate kinase (Accession No. P26512); the gene *lysE* coding for lysine export (DE-A-195 48 222); the gene *horn* coding for homoserine dehydrogenase (EP-A 0131171); the gene *ilvA* coding for threonine dehydratase (Möckel et al., Journal of Bacteriology (1992) 8065-8072)) or the allele *ilvA (Fbr)* coding for a feedback-resistant threonine dehydratase (Möckel et al., (1994) Molecular Microbiology 13: 833-842); the gene *ilvBN* coding for acetohydroxy acid synthase (EP-B 0356739); the gene *ilvD* coding for dihydroxy acid dehydratase (Sahm and Eggeling (1999) Applied and Environmental Microbiology 65: 1973-1979); and the gene *zwa1* coding for the Zwa1 protein (DE: 19959328.0, DSM 13115).

Furthermore it may be advantageous for the production of L-amino acids also to attenuate, in particular to reduce, the expression of one or more genes selected from the group: the gene pck coding for phosphoenol pyruvate carboxykinase (DE 199 50 409.1; DSM 13047); the gene pgi coding for glucose-6-phosphate isomerase (U.S. Pat. No. 6,586,214; DSM 12969); the gene poxB coding for pyruvate oxidase (DE: 1995 1975.7; DSM 13114); and the gene zwa2 coding for the Zwa2 protein (DE: 19959327.2, DSM 13113).

In addition, it may furthermore be advantageous, for the production of amino acids, in particular L-lysine, to eliminate undesirable side reactions, (Nakayama: "Breeding of Amino Acid Producing Micro-organisms", in: Overproduction of Microbial Products, Krumphanzl, Sikyta, Vanek (eds.), Academic Press, London, UK, 1982).

The promoter according to the invention can thus be used in each case for overexpressing or underexpressing the target gene in *C. glutamicum.*

### Linkers

The target gene is positioned downstream of the promoter polynucleotide according to the invention, *i.e.* at the 3' end, such that both polynucleotides are functionally linked to one another either directly or by means of a linker oligonucleotide or linker polynucleotide. Preference is given to the promoter and the target gene being functionally linked to one another by means of a linker oligonucleotide or linker polynucleotide. Said linker oligonucleotide or linker polynucleotide consists of deoxyribonucleotides.

In this context, the expression "functionally linked to one another directly" means that the nucleotide at the 3' end of SEQ ID NO:1, SEQ ID NO:2, SEQ ID NO:3, SEQ ID NO:4, SEQ ID NO:5, SEQ ID NO:6, SEQ ID NO:7, or SEQ ID NO:8 is linked directly to the first nucleotide of the start codon of a target gene. This results in "leaderless" mRNAs which start immediately with the 5'-terminal AUG start codon and therefore do not have any other translation initiation signals.

In this context, the expression "functionally linked to one another by means of a linker oligonucleotide" means that the nucleotide at the 3' end of SEQ ID NO:1, SEQ ID NO:2, SEQ ID NO:3, SEQ ID NO:4, SEQ ID NO:5, SEQ ID NO:6, SEQ ID NO:7, or SEQ ID NO:8 is linked by an oligonucleotide of 1, 2, 3, 4 or 5 nucleotides in length to the first nucleotide of the start codon of a target gene.

In this context, the expression "functionally linked to one another by means of a linker polynucleotide" means that the nucleotide at the 3' end of SEQ ID NO:1, SEQ ID NO:2, SEQ ID NO:3, SEQ ID NO:4, SEQ ID NO:5, SEQ ID NO:6, SEQ ID NO:7, or SEQ ID NO:8 is linked by a polynucleotide of from 6 to no more than 600 nucleotides in length to the first nucleotide of the start codon of a target gene.

In this context, the expression "functionally linked to one another" means that the target gene is bound to the promoter polynucleotide according to the invention in such a way that transcription of the target gene and translation of the synthesized RNA are ensured.

Depending on the technical requirement, the linker polynucleotide is:
6 - 600, 6 - 500, 6 - 400, 6 - 300, 6 - 200, 6 - 180, 6 -160, 6 - 140, 6 - 120, 6 - 100, 6 - 80, 6 - 60, 6 - 50, 6 - 40, 6 - 30, 6 - 28, 6 - 27, 6 - 26, 6 - 25; or
8 - 600, 8 - 500, 8 - 400, 8 - 300, 8 - 200, 8 - 180, 8 -160, 8 - 140, 8 - 120, 8 - 100, 8 - 80, 8 - 60, 8 - 50, 8 - 40, 8 - 30, 8 - 28, 8 - 27, 8 - 26, 8 - 25; or
10 - 600, 10 - 500, 10 - 400, 10 - 300, 10 - 200, 10 - 180, 10 - 160, 10 - 140, 10 - 120, 10 - 100, 10 - 80, 10 - 60, 10 - 50, 10 - 40, 10 - 30, 10 - 28, 10 - 27, 10 - 26, 10 -25; or
12 - 600, 12 - 500, 12 - 400, 12 - 300, 12 - 200, 12 - 180, 12 - 160, 12 - 140, 12 - 120, 12 - 100, 12 - 80, 12 - 60, 12 - 50, 12 - 40, 12 - 30, 12 - 28, 12 - 27, 12 - 26, 12 -25; or
14 - 600, 14 - 500, 14 - 400, 14 - 300, 14 - 200, 14 - 180, 14 - 160, 14 - 140, 14 - 120, 14 - 100, 14 - 80, 14 - 60, 14 - 50, 14 - 40, 14 - 30, 14 - 28, 14 - 27, 14 - 26, 14 -20; or
16 - 600, 16 - 500, 16 - 400, 16 - 300, 16 - 200, 16 - 180, 16 - 160, 16 - 140, 16 - 120, 16 - 100, 16 - 80, 16 - 60, 16 - 50, 16 - 40, 16 - 30, 16 - 28, 16 - 27, 16 - 26, 16 -25; or
18 - 600, 18 - 500, 18 - 400, 18 - 300, 18 - 200, 18 - 180, 18 - 160, 18 - 140, 18 - 120, 18 - 100, 18 - 80, 18 - 60, 18 - 50, 18 - 40, 18 - 30, 18 - 28, 18 - 27, 18 - 26, 18 -25; or
20 - 600, 20 - 500, 20 - 400, 20 - 300, 20 - 200, 20 - 180, 20 - 160, 20 - 140, 20 - 120, 20 - 100, 20 - 80, 20 - 60, 20 - 50, 20 - 40, 20 - 30, 20 - 28, 20 - 27, 20 - 26, 20 -25 nucleotides in length.

In particularly preferred embodiments, the linker polynucleotide is 20, 21, 22, 23, 24, or 25 nucleotides in length because this produces preferably functional constructs.

The invention further relates accordingly to an isolated promoter polynucleotide, essentially consisting of a polynucleotide of SEQ ID NO:1, SEQ ID NO:2, SEQ ID NO:3, SEQ ID NO:4, SEQ ID NO:5, SEQ ID NO:6, SEQ ID NO:7, or SEQ ID NO:8, which, via the nucleotide at its 3' end, is functionally linked, directly or by means of a linker polynucleotide which ensures translation of RNA, to a target gene which contains at its 5' end an ATG or GTG start codon and codes for one or more polypeptide(s). Preference is given to the promoter and target gene being functionally linked to one another by means of a linker polynucleotide.

The invention furthermore also relates to an isolated polynucleotide, essentially consisting of a polynucleotide of SEQ ID NO:1, SEQ ID NO:2, SEQ ID NO:3, SEQ ID NO:4, SEQ ID NO:5, SEQ ID NO:6, SEQ ID NO:7, or SEQ ID NO:8, which, via the nucleotide at its 3' end, is functionally linked to a linker oligonucleotide.

In addition, the invention furthermore relates to an isolated polynucleotide, essentially consisting of a polynucleotide of SEQ ID NO:1, SEQ ID NO:2, SEQ ID NO:3, SEQ ID NO:4, SEQ ID NO:5, SEQ ID NO:6, SEQ ID NO:7, or SEQ ID NO:8, which, via the nucleotide at its 3' end, is functionally linked to a linker polynucleotide which ensures translation of RNA.

In this context, the term "essentially" means that a polynucleotide of no more than 1,000, no more than 800, no more than 700, no more than 600, no more than 500, or no more than 400 nucleotides in length has been added to the 5' end of the polynucleotide of SEQ ID NO:1, SEQ ID NO:2, SEQ ID NO:3, SEQ ID NO:4, SEQ ID NO:5, SEQ ID NO:6, SEQ ID NO:7, or SEQ ID NO:8 and a polynucleotide of no more than 1,000, no more than 800, no more than 700, no more than 600, no more than 500, or no more than 400 nucleotides in length has been added to the 3' of the target gene, or a polynucleotide of no more than 15,000, no more than 10,000, no more than 7,500, no more than 5,000, no more than 2,500, no more than 1,000, no more than 800, no more than 700, no more than 600, no more than 500, or no more than 400 nucleotides in length has been added to the 3' end of the linker oligo- or polynucleotide.

Any useful combination of the features from the preceding three lists of polynucleotides is in accordance with the invention here. "Useful combination" means, for example, a combination of features which results in an efficient recombination being carried out. The use of additions of the same length flanking a DNA region to be replaced facilitates the transfer of the region by homologous recombination in the experimental procedure. Relatively long flanking homologous regions are advantageous for efficient recombination between circular DNA molecules but cloning of the replacement vector is made more difficult with increasing length of the flanks (Wang et al., Molecular Biotechnology 32:43-53 (2006)).

In addition, the flank at the 3' end of the linker oligo- or polynucleotide increases in length to no more than 15,000 nucleotides when the 3' end is functionally linked to a target gene which contains at its 5' end an ATG or GTG start codon and codes for one or more polypeptide(s).

These particularly preferred embodiments of the linker polynucleotide ensure translation of RNA in an advantageous manner.

To facilitate chemical linking between the polynucleotide according to the invention having the nucleotide sequence of SEQ ID NO:1, SEQ ID NO:2, SEQ ID NO:3, SEQ ID NO:4, SEQ ID NO:5, SEQ ID NO:6, SEQ ID NO:7, or SEQ ID NO:8, the linker polynucleotide which ensures translation of RNA, and the target gene coding for one or more polypeptide(s), which has an ATG or GTG start codon at its 5' end, functional nucleotide sequences required for cloning may be incorporated into said polynucleotides at their 5' and 3' ends and are at least partially retained even after said cloning.

The term "functional nucleotide sequence required for cloning" here represents any REII (type II restriction endonuclease) cleavage site present, whose sequence normally consists of from 4 to 8 nucleotides.

In addition, it should be mentioned here that site-specific mutagenesis by means of mutagenesis primers or a *de novo* gene synthesis (*e.g.* by GENEART AG (Regensburg, Germany)) of the nucleotide sequences to remove cleavage sites for restriction endonucleases may introduce silent mutations into the sequence in order to enable said cleavage sites to be used advantageously for subsequent cloning steps.

The polynucleotide resulting from the promoter according to the invention being functionally linked to the linker polynucleotide which ensures translation of RNA is also referred to as expression unit herein below.

### Expression

The invention furthermore relates to the use of the promoter according to the invention or of the expression unit according to the invention for expressing target genes or polynucleotides in microorganisms. The promoter according to the invention or the expression unit according to the invention ensures transcription and translation of the synthesized RNA, preferably mRNA, into a polypeptide. As used herein, the term "host cell" refers to a transformed cell of a microorganism.

The present disclosure, provides for, and includes, transformed host cells comprising the recombinant nucleic acids and recombinant vectors described in detail above. The present disclosure further provides for, and includes, host cells transformed with two recombinant nucleic acids. In an embodiment, the host cells are transformed with three recombinant nucleic acids. As provided above, the nucleic acids may be selected from biosynthetic pathways based on the overall effect on the yield of the desired product. There is no practical limit the the number of recombinant nucleic acids that may be incorporated into the host cells of the present specification. Expression is preferably carried out in microorganisms of the genus *Corynebacterium.* Preference is given to strains within the genus *Corynebacterium* which are based on the following species: *C. efficiens,* with the deposited type strain being DSM44549; *C. glutamicum,* with the deposited type strain being ATCC13032; and *C. ammoniagenes,* with the deposited type strain being ATCC6871. Very particular preference is given to the species *C. glutamicum.* In this way it is possible to express polynucleotides that code for polypeptides having a property, preferably enzyme activity, which are not present or detectable in the corresponding host. Thus, for example, Yukawa et al. describe expression of Escherichia coli genes for utilizing D-xylose in C. glutamicum R under the control of the constitutive Ptrc promoter (Applied Microbiology and Biotechnology 81, 691-699 (2008)).

The present specification provides for, and includes *C. glutamicum* having two or more genes of a biosynthetic pathway under the control of the promoter polynucleotide sequences described above. In various embodiments, one or more target genes are placed under the control of a promoter polynucleotide sequence having as sequence of SEQ ID NOs:1 to 8 as described above. In other embodiments, one or more target genes are placed under the control of a promoter polynucleotide sequence having as sequence of SEQ ID NOs:1, 5 or 7 as described above.

In certain embodiments according to the present specification, *C. glutamicum* host cells have two target genes under the control of the promoters having sequences of SEQ ID NOs:1 to 8. In certain other embodiments according to the present specification, *C. glutamicum* host cells have two target genes under the control of the promoters having sequences of SEQ ID NOs:1, 5 or 7. Using homologous recombination, the promoters of the present disclosure replace the endogenous promoter and endogenous sequence to prepare a promoter functionally linked to a heterologous gene. One of ordinary skill in the art would recognize that the recombination results in a replacement of the endogenous promoter while retaining the gene in its native locus. Specific non-limiting examples are illustrated below in Table 8. Multiple promoter-heterologous target pairs (e.g., promoter cassettes) can be readily incorporated into the genome of a host cell. In an embodiment, the promoter cassettes can be incorporated into host cells sequentially. In certain embodiments, the recombinant vectors of the present disclosure provide for two or more different promoter cassettes in a single construct. The present specification provides no practical limit to the number of promoter replacements that can be developed using the described methods.

In an embodiment the host cell is a transgenic *C. glutamicum* host cell comprising the promoter cassettes Pcg0007-lysA and Pcg3121-pgi. In an embodiment the host cell is a transgenic *C. glutamicum* host cell comprising the promoter cassettes Pcg1860-pyc and Pcg0007-zwf. In an embodiment the host cell is a transgenic *C. glutamicum* host cell comprising the promoter cassettes Pcg0007-lysA and Pcg0007-zwf. In an embodiment the host cell is a transgenic *C. glutamicum* host cell comprising the promoter cassettes Pcg3121-pck and Pcg0007-zwf. In an embodiment the host cell is a transgenic *C. glutamicum* host cell comprising the promoter cassettes Pcg0007_39-ppc and Pcg0007-zwf. In an embodiment the host cell is a transgenic *C. glutamicum* host cell comprising the promoter cassettes Pcg3121-pck and Pcg3121-pgi. In an embodiment the host cell is a transgenic *C. glutamicum* host cell comprising the promoter cassettes Pcg3381-ddh and Pcg0007-zwf. In an embodiment the host cell is a transgenic *C. glutamicum* host cell comprising the promoter cassettes Pcg0007_265-dapB and Pcg0007-zwf. In an embodiment the host cell is a transgenic *C. glutamicum* host cell comprising the promoter cassettes Pcg0007-zwf and Pcg3121-pgi. In an embodiment the host cell is a transgenic *C. glutamicum* host cell comprising the promoter cassettes Pcg3381-ddh and Pcg3121-pgi. In an embodiment the host cell is a transgenic *C. glutamicum* host cell comprising the promoter cassettes Pcg3121-pgi and Pcg1860-pyc. In an embodiment the host cell is a transgenic *C. glutamicum* host cell comprising the promoter cassettes Pcg1860-pyc and Pcg0007_265-dapB. In an embodiment the host cell is a transgenic *C. glutamicum* host cell comprising the promoter cassettes Pcg1860-pyc and Pcg0007-lysA. In an embodiment the host cell is a transgenic *C. glutamicum* host cell comprising the promoter cassettes Pcg1860-asd and Pcg0007-zwf. In an embodiment the host cell is a transgenic *C. glutamicum* host cell comprising the promoter cassettes Pcg0007_265-dapB and Pcg3121-pgi. In an embodiment the host cell is a transgenic *C. glutamicum* host cell comprising the promoter cassettes Pcg1860-pyc and Pcg1860-asd. In an embodiment the host cell is a transgenic *C. glutamicum* host cell comprising the promoter cassettes Pcg3381-aspB and Pcg1860-pyc. In an embodiment the host cell is a transgenic *C. glutamicum* host cell comprising the promoter cassettes Pcg3381-fbp and Pcg1860-pyc. In an embodiment the host cell is a transgenic *C. glutamicum* host cell comprising the promoter cassettes Pcg3381-ddh and Pcg3381-fbp. In an embodiment the host cell is a transgenic *C. glutamicum* host cell comprising the promoter cassettes Pcg0755-ptsG and Pcg3121-pgi. In an embodiment the host cell is a transgenic *C. glutamicum* host cell comprising the promoter cassettes Pcg1860-pyc and Pcg3121-pck. In an embodiment the host cell is a transgenic *C. glutamicum* host cell comprising the promoter cassettes Pcg1860-asd and Pcg3121-pgi. In an embodiment the host cell is a transgenic *C. glutamicum* host cell comprising the promoter cassettes Pcg1860-asd and Pcg3381-fbp. In an embodiment the host cell is a transgenic C. *glutamicum* host cell comprising the promoter cassettes Pcg0007_39-lysE and Pcg3381-fbp. In an embodiment the host cell is a transgenic *C. glutamicum* host cell comprising the promoter cassettes Pcg3381-fbp and Pcg0007-lysA. In an embodiment the host cell is a transgenic *C. glutamicum* host cell comprising the promoter cassettes Pcg0007_39-lysE and Pcg1860-pyc. In an embodiment the host cell is a transgenic *C. glutamicum* host cell comprising the promoter cassettes Pcg3121-pgi and Pcg3381-fbp. In an embodiment the host cell is a transgenic *C. glutamicum* host cell comprising the promoter cassettes Pcg3121-pck and Pcg0007-lysA. In an embodiment the host cell is a transgenic *C. glutamicum* host cell comprising the promoter cassettes Pcg0007-lysA and Pcg0007 _265-dapB. In an embodiment the host cell is a transgenic *C. glutamicum* host cell comprising the promoter cassettes Pcg0007 265-dapB and Pcg1860-asd. In an embodiment the host cell is a transgenic *C. glutamicum* host cell comprising the promoter cassettes Pcg3121-pgi and Pcg0007 _265-dapD. In an embodiment the host cell is a transgenic *C. glutamicum* host cell comprising the promoter cassettes Pcg0007-lysA and Pcg3381-ddh. In an embodiment the host cell is a transgenic *C. glutamicum* host cell comprising the promoter cassettes Pcg3121-pck and Pcg1860-asd. In an embodiment the host cell is a transgenic *C. glutamicum* host cell comprising the promoter cassettes Pcg0007-lysA and Pcg1860-asd. In an embodiment the host cell is a transgenic *C. glutamicum* host cell comprising the promoter cassettes Pcg3121-pck and Pcg0007_265-dapB. In an embodiment the host cell is a transgenic *C. glutamicum* host cell comprising the promoter cassettes Pcg3381-ddh and Pcg1860-asd. In an embodiment the host cell is a transgenic *C. glutamicum* host cell comprising the promoter cassettes Pcg0007_39-ppc and Pcg1860-asd. In an embodiment the host cell is a transgenic *C. glutamicum* host cell comprising the promoter cassettes Pcg0007_39-ppc and Pcg0007-lysA. In an embodiment the host cell is a transgenic *C. glutamicum* host cell comprising the promoter cassettes Pcg3381-ddh and Pcg0007 _265-dapB. In an embodiment the host cell is a transgenic *C. glutamicum* host cell comprising the promoter cassettes Pcg0007 265-dapB and Pcg3381-fbp. In an embodiment the host cell is a transgenic *C. glutamicum* host cell comprising the promoter cassettes Pcg0007_39-ppc and Pcg0007 _265-dapB. In an embodiment the host cell is a transgenic *C. glutamicum* host cell comprising the promoter cassettes Pcg3381-aspB and Pcg3121-pck. In an embodiment the host cell is a transgenic *C. glutamicum* host cell comprising the promoter cassettes Pcg0007 265-dapB and Pcg0007 _265-dapD. In an embodiment the host cell is a transgenic *C. glutamicum* host cell comprising the promoter cassettes Pcg0007_39-lysE and Pcg3381-aspB. In an embodiment the host cell is a transgenic *C. glutamicum* host cell comprising the promoter cassettes Pcg0007_39-lysE and Pcg0007 _265-dapD. In an embodiment the host cell is a transgenic *C. glutamicum* host cell comprising the promoter cassettes Pcg3381-aspB and Pcg0007 _265-dapB. In an embodiment the host cell is a transgenic *C. glutamicum* host cell comprising the promoter cassettes Pcgl1860-asd and Pcg0007 _265-dapD. In an embodiment the host cell is a transgenic *C. glutamicum* host cell comprising the promoter cassettes Pcg3381-aspB and Pcg0007-lysA. In an embodiment the host cell is a transgenic *C. glutamicum* host cell comprising the promoter cassettes Pcg3381-aspB and Pcg3381-ddh. In an embodiment the host cell is a transgenic *C. glutamicum* host cell comprising the promoter cassettes Pcg0755-ptsG and Pcg1860-pyc. In an embodiment the host cell is a transgenic *C. glutamicum* host cell comprising the promoter cassettes Pcg0755-ptsG and Pcg3381-fbp. In an embodiment the host cell is a transgenic *C. glutamicum* host cell comprising the promoter cassettes Pcg0007-zwf and Pcg3381-fbp. In an embodiment the host cell is a transgenic *C. glutamicum* host cell comprising the promoter cassettes Pcg0755-ptsG and Pcg0007_265-dapD.

The present disclosure provides for, and includes, host cells having three or more promoter cassettes as described above. In an embodment, the host cell includes the Pcg0007_39-zwf, Pcg0007_39-lysA and Pcg1860-pyc promoter cassettes. In an embodiment, the host cell is a *C. glutamicum* host cell.

The promoter according to the invention or the expression unit according to the invention is furthermore used for improving the performance characteristics of microorganisms, which can include, for example, yield, titer, productivity, by-product elimination, tolerance to process excursions, optimal growth temperature and growth rate. In some embodiments, the promoter according to the invention or the expression unit according to the invention is used for up-regulating a target gene in a microorganism (overexpression). Overexpression generally means an increase in the intracellular concentration or activity of a ribonucleic acid, a protein (polypeptide) or an enzyme in comparison with the starting strain (parent strain) or wild-type strain, if the latter is the starting strain. In some embodiments, the promoter according to the invention or the expression unit according to the invention is used for down-regulating a target gene in a microorganism (underexpression). Underexpression generally means an decrease in the intracellular concentration or activity of a ribonucleic acid, a protein (polypeptide) or an enzyme in comparison with the starting strain (parent strain) or wild-type strain, if the latter is the starting strain. In some embodiments, a combination of promoters and/or expression units according to the invention are used for regulating expression of more than one target gene in a microorganism, wherein each target gene is either up-regulated or down-regulated. In some embodiments the target genes up- or down-regulated by the combination of promoters and/or expression units are part of the same metabolic pathway. In some embodiments the target genes up- or down-regulated by the combination of promoters and/or expression units are not part of the same metabolic pathway.

The promoters described herein can be used in combination with other methods very well-known in the art for attenuating (reducing or eliminating) the intracellular activity of one or more enzymes (proteins) in a microorganism which are coded by the corresponding DNA, for example by using a weak promoter or using a gene, or allele, which codes for a corresponding enzyme with a low activity, or inactivates the corresponding gene or enzyme (protein), and optionally combining these measures.

The reduction in gene expression can take place by suitable culturing or by genetic modification (mutation) of the signal structures of gene expression. Signal structures of gene expression are, for example, repressor genes, activator genes, operators, promoters, attenuators, ribosome binding sites, the start codon and terminators. The expert can find information on this *e.g*. in the patent application WO 96/15246, in Boyd and Murphy (Journal of Bacteriology 170: 5949 (1988)), in Voskuil and Chambliss (Nucleic Acids Research 26: 3548 (1998), in Jensen and Hammer (Biotechnology and Bioengineering 58: 191 (1998)), in Patek et al. (Microbiology 142: 1297 (1996)), Vaš̌̌icová et al. (Journal of Bacteriology 181: 6188 (1999)) and in known textbooks of genetics and molecular biology, such as *e.g.* the textbook by Knippers ("Molekulare Genetik [Molecular Genetics]", 6th edition, Georg Thieme Verlag, Stuttgart, Germany, 1995) or that by Winnacker ("Gene und Klone [Genes and Clones]", VCH Verlagsgesellschaft, Weinheim, Germany, 1990).

Mutations which lead to a change or reduction in the catalytic properties of enzyme proteins are known from the prior art; examples which may be mentioned are the works by Qiu and Goodman (Journal of Biological Chemistry 272: 8611-8617 (1997)), Sugimoto et al. (Bioscience Biotechnology and Biochemistry 61: 1760-1762 (1997)) and Möckel ("Die Threonindehydratase aus Corynebacterium glutamicum: Aufhebung der allosterischen Regulation und Struktur des Enzyms [Threonine dehydratase from Corynebacterium glutamicum: Cancelling the allosteric regulation and structure of the enzyme]", Reports from the Jülich Research Centre, Jül-2906, ISSN09442952, Jülich, Germany, 1994). Comprehensive descriptions can be found in known textbooks of genetics and molecular biology, such as *e.g.* that by Hagemann ("Allgemeine Genetik [General Genetics]", Gustav Fischer Verlag, Stuttgart, 1986).

Possible mutations are transitions, transversions, insertions and deletions. Depending on the effect of the amino acid exchange on the enzyme activity, missense mutations or nonsense mutations are referred to. Insertions or deletions of at least one base pair in a gene lead to frame shift mutations, as a consequence of which incorrect amino acids are incorporated or translation is interrupted prematurely. Deletions of several codons typically lead to a complete loss of the enzyme activity. Instructions on generation of such mutations are prior art and can be found in known textbooks of genetics and molecular biology, such as *e.g*. the textbook by Knippers ("Molekulare Genetik [Molecular Genetics]", 6th edition, Georg Thieme Verlag, Stuttgart, Germany, 1995), that by Winnacker ("Gene und Klone [Genes and Clones]", VCH Verlagsgesellschaft, Weinheim, Germany, 1990) or that by Hagemann ("Allgemeine Genetik [General Genetics]", Gustav Fischer Verlag, Stuttgart, 1986). A common method of mutating genes of *C. glutamicum* is the method of gene disruption and gene replacement described by Schwarzer and Pühler (Bio/Technology 9, 84-87 (1991)).

In the method of gene disruption a central part of the coding region of the gene of interest is cloned in a plasmid vector which can replicate in a host (typically *E. coli*), but not in *C. glutamicum.* Possible vectors are, for example, pSUP301 (Simon et al., Bio/Technology 1, 784-791 (1983)), pK18mob or pK19mob (Schäfer et al., Gene 145, 69-73 (1994)), pK18mobsacB or pK19mobsacB (Jager et al., Journal of Bacteriology 174: 5462-65 (1992)), pGEM-T (Promega corporation, Madison, Wis., USA), pCR2.1-TOPO (Shuman (1994). Journal of Biological Chemistry 269:32678-84; U.S. Pat. No. 5,487,993), pCR®Blunt (Invitrogen, Groningen, Holland; Bernard et al., Journal of Molecular Biology, 234: 534-541 (1993)) or pEMl (Schrumpf et al, 1991, Journal of Bacteriology 173:4510-4516). The plasmid vector which contains the central part of the coding region of the gene is then transferred into the desired strain of *C. glutamicum* by conjugation or transformation. The method of conjugation is described, for example, by Schäfer et al. (Applied and Environmental Microbiology 60, 756-759 (1994)). Methods for transformation are described, for example, by Thierbach et al. (Applied Microbiology and Biotechnology 29, 356-362 (1988)), Dunican and Shivnan (Bio/Technology 7, 1067-1070 (1989)) and Tauch et al. (FEMS Microbiological Letters 123, 343-347 (1994)). After homologous recombination by means of a "cross-over" event, the coding region of the gene in question is interrupted by the vector sequence and two incomplete alleles are obtained, one lacking the 3' end and one lacking the 5' end. This method has been used, for example, by Fitzpatrick et al. (Applied Microbiology and Biotechnology 42, 575-580 (1994)) to eliminate the recA gene of *C. glutamicum.*

In the method of gene replacement, a mutation, such as *e.g.* a deletion, insertion or base exchange, is established *in vitro* in the gene of interest. The allele prepared is in turn cloned in a vector which is not replicative for *C. glutamicum* and this is then transferred into the desired host of *C. glutamicum* by transformation or conjugation. After homologous recombination by means of a first "cross-over" event which effects integration and a suitable second "cross-over" event which effects excision in the target gene or in the target sequence, the incorporation of the mutation or of the allele is achieved. This method was used, for example, by Peters-Wendisch (Microbiology 144, 915-927 (1998)) to eliminate the pyc gene of *C. glutamicum* by a deletion.

The promoters described herein can be used in combination with other methods very well-known in the art for raising (enhancing) the intracellular activity of one or more enzymes in a microorganism that are coded by the corresponding DNA, by for example increasing the number of copies of the gene or genes, using a strong promoter, or using a gene that codes for a corresponding enzyme having a high activity, and optionally combining these measures.

In order to achieve an overexpression the number of copies of the corresponding genes can be increased, or alternatively the promoter and regulation region or the ribosome binding site located upstream of the structure gene can be mutated. Expression cassettes that are incorporated upstream of the structure gene act in the same way. By means of inducible promoters it is in addition possible to increase the expression in the course of the enzymatic amino acid production. The expression is similarly improved by measures aimed at prolonging the lifetime of the m-RNA. Furthermore, the enzyme activity is also enhanced by preventing the degradation of the enzyme protein. The genes or gene constructs may either be present in plasmids having different numbers of copies, or may be integrated and amplified in the chromosome. Alternatively, an overexpression of the relevant genes may furthermore be achieved by altering the composition of the media and the culture conditions.

The person skilled in the art can find details on the above in, *inter alia,* Martin et al. (Bio/Technology 5, 137-146 (1987)), in Guerrero et al. (Gene 138, 35-41 (1994)), Tsuchiya and Morinaga (Bio/Technology 6, 428-430 (1988)), in Eikmanns et al. (Gene 102, 93-98 (1991)), in European Patent Specification 0 472 869, in U.S. Pat. No. 4,601,893, in Schwarzer and Pühler (Bio/Technology 9, 84-87 (1991), in Reinscheid et al. (Applied and Environmental Microbiology 60, 126-132 (1994)), in LaBarre et al. (Journal of Bacteriology 175, 1001-1007 (1993)), in Patent Application WO 96/15246, in Malumbres et al. (Gene 134, 15-24 (1993)), in Japanese laid open Specification JP-A-10-229891, in Jensen and Hammer (Biotechnology and Bioengineering 58, 191-195 (1998)), in Makrides (Microbiological Reviews 60:512-538 (1996)) and in known textbooks on genetics and molecular biology.

Genes may be overexpressed for example by means of episomal plasmids. Suitable plasmids are those that are replicated in coryneform bacteria. Numerous known plasmid vectors, such as for example pZ1 (Menkel et al., Applied and Environmental Microbiology (1989) 64: 549-554), pEKEx1 (Eikmanns et al., Gene 102:93-98 (1991)) or pHS2-1 (Sonnen et al., Gene 107:69-74 (1991)) are based on the cryptic plasmids pHM1519, pBL1 or pGA1. Other plasmid vectors, such as for example those based on pCG4 (U.S. Pat. No. 4,489,160), or pNG2 (Serwold-Davis et al., FEMS Microbiology Letters 66, 119-124 (1990)), or pAG1 (U.S. Pat. No. 5,158,891) may be used in a similar way.

Furthermore, also suitable are those plasmid vectors with the aid of which the process of gene amplification by integration in the chromosome can be employed, such as has been described for example by Reinscheid et al. (Applied and Environmental Microbiology 60, 126-132 (1994)) for the duplication and amplification of the hom-thrB operon. In this method the complete gene is cloned into a plasmid vector that can replicate in a host (typically *E. coli*) but not in C. *glutamicum.* Suitable vectors are for example pSUP301 (Simon et al., Bio/Technology 1, 784-791 (1983)), pK18mob or pK19 mob (Schäfer et al., Gene 145, 69-73 (1994)), pGEM-T (Promega Corporation, Madison, Wis., USA), pCR2.1-TOPO (Shuman (1994). Journal of Biological Chemistry 269:32678-84; U.S. Pat. No. 5,487,993), pCR®Blunt (Invitrogen, Groningen, Netherlands; Bernard et al., Journal of Molecular Biology, 234: 534-541 (1993)), pEM1 (Schrumpf et al, 1991, Journal of Bacteriology 173:4510-4516) or pBGS8 (Spratt et al., 1986, Gene 41: 337-342). The plasmid vector that contains the gene to be amplified is then transferred by conjugation or transformation into the desired strain of *C. glutamicum.* The method of conjugation is described for example in Schäfer et al. (Applied and Environmental Microbiology 60, 756-759 (1994)). Transformation methods are described for example in Thierbach et al. (Applied Microbiology and Biotechnology 29, 356-362 (1988)), Dunican and Shivnan (Bio/Technology 7, 1067-1070 (1989)) and Tauch et al. (FEMS Microbiological Letters 123, 343-347 (1994)). After homologous recombination by means of a crossover event, the resulting strain contains at least two copies of the relevant gene.

Methods of regulating, *i.e.,* either increasing or decreasing, gene expression include recombinant methods in which a microorganism is produced using a DNA molecule provided *in vitro.* Such DNA molecules comprise, for example, promoters, expression cassettes, genes, alleles, coding regions, *etc.* They are introduced into the desired microorganisms by methods of transformation, conjugation, transduction or similar methods.

In the case of the present invention, the promoters are preferably a polynucleotide of SEQ ID NO:1, SEQ ID NO:2, SEQ ID NO:3, SEQ ID NO:4, SEQ ID NO:5, SEQ ID NO:6, SEQ ID NO:7, or SEQ ID NO:8, and the expression cassettes are preferably a polynucleotide of SEQ ID NO:1, SEQ ID NO:2, SEQ ID NO:3, SEQ ID NO:4, SEQ ID NO:5, SEQ ID NO:6, SEQ ID NO:7, or SEQ ID NO:8 which, via the nucleotide at its 3' end, are functionally linked to a linker polynucleotide which ensures translation of RNA.

The measures of overexpression using the promoter according to the invention or the expression unit according to the invention increase the activity or concentration of the corresponding polypeptide usually by at least 10%, 25%, 50%, 75%, 100%, 150%, 200%, 300%, 400% or 500%, preferably by no more than 1,000%, 2,000%, 4,000%, 10,000% or 20,000%, based on the activity or concentration of said polypeptide in the strain prior to the measure resulting in overexpression.

The extent of expression or overexpression may be established by measuring the amount of mRNA transcribed from the gene, by determining the amount of polypeptide and by determining enzyme activity.

The amount of mRNA may be determined *inter alia* by using the methods of "Northern Blotting" and of quantitative RT-PCR. Quantitative RT-PCR involves reverse transcription which precedes the polymerase chain reaction. For this, the LightCycler™ System from Roche Diagnostics (Boehringer Mannheim GmbH, Roche Molecular Biochemicals, Mannheim, Germany) may be used, as described in Jungwirth et al. (FEMS Microbiology Letters 281, 190-197 (2008)), for example. The concentration of the protein may be determined via 1- and 2-dimensional protein gel fractionation and subsequent optical identification of the protein concentration using appropriate evaluation software in the gel. A customary method of preparing protein gels for coryneform bacteria and of identifying said proteins is the procedure described by Hermann et al. (Electrophoresis, 22:1712-23 (2001)). The protein concentration may likewise be determined by Western-Blot hybridization using an antibody specific for the protein to be detected (Sambrook et al., Molecular cloning: a laboratory manual. 2nd Ed. Cold Spring Harbor Laboratory Press, Cold Spring Harbor, N.Y., 1989) and subsequent optical evaluation using appropriate software for concentration determination (Lohaus and Meyer (1998) Biospektrum 5:32-39; Lottspeich, Angewandte Chemie 321: 2630-2647 (1999)). The statistical significance of the data collected is determined by means of a T test (Gosset, Biometrika 6(1): 1-25 (1908)).

The measure of overexpressing target genes using the promoter according to the invention may be combined in a suitable manner with further overexpression measures. Overexpression is achieved by a multiplicity of methods available in the prior art. These include increasing the copy number in addition to modifying the nucleotide sequences which direct or control expression of the gene. The copy number may be increased by means of plasmids which replicate in the cytoplasm of the microorganism. To this end, an abundance of plasmids are described in the prior art for very different groups of microorganisms, which plasmids can be used for setting the desired increase in the copy number of the gene. Plasmids suitable for the genus *Corynebacterium* are described, for example, in Tauch et al. (Journal of Biotechnology 104 (1-3), 27-40, (2003)), and in Stansen et al. (Applied and Environmental Microbiology 71, 5920-5928 (2005)).

The copy number may furthermore be increased by at least one (1) copy by introducing further copies into the chromosome of the microorganism. Methods suitable for the genus *Corynebacterium* are described, for example, in the patents WO 03/014330, WO 03/040373 and WO 04/069996.

Gene expression may furthermore be increased by positioning a plurality of promoters upstream of the target gene or functionally linking them to the gene to be expressed and achieving increased expression in this way. Examples of this are described in the patent WO 2006/069711.

Transcription of a gene is controlled, where appropriate, by proteins which suppress (repressor proteins) or promote (activator proteins) transcription. Accordingly, overexpression can likewise be achieved by increasing the expression of activator proteins or reducing or switching off the expression of repressor proteins or else eliminating the binding sites of the repressor proteins. The rate of elongation is influenced by the codon usage, it being possible to enhance translation by utilizing codons for transfer RNAs (tRNAs) which are frequent in the starting strain. Moreover, replacing a start codon with the ATG codon most frequent in many microorganisms (77% in *E. coli*) may considerably improve translation, since, at the RNA level, the AUG codon is two to three times more effective than the codons GUG and UUG, for example (Khudyakov et al., FEBS Letters 232(2):369-71(1988); Reddy et al., Proceedings of the National Academy of Sciences of the USA 82(17):5656-60 (1985)). It is also possible to optimize the sequences surrounding the start codon because synergistic effects between the start codon and the flanking regions have been described (Stenström et al., Gene 273(2):259-65 (2001); Hui et al., EMBO Journal 3(3):623-9 (1984)).

Instructions for handling DNA, digestion and ligation of DNA, transformation and selection of transformants can be found *inter alia* in the known manual by Sambrook et al. "Molecular Cloning: A Laboratory Manual, Second Edition (Cold Spring Harbor Laboratory Press, 1989).

The invention also relates to vectors comprising the polynucleotides according to the invention.

Kirchner and Tauch (Journal of Biotechnology 104:287-299 (2003)) describe a selection of vectors to be used in *C. glutamicum.*

Homologous recombination using the vectors according to the invention allows DNA segments on the chromosome to be replaced with polynucleotides according to the invention which are transported into the cell by the vector. For efficient recombination between the circular DNA molecule of the vector and the target DNA on the chromosome, the DNA region to be replaced with the polynucleotide according to the invention is provided at the ends with nucleotide sequences homologous to the target site which determine the site of integration of the vector and of replacement of the DNA.

Thus the promoter polynucleotide according to the invention may: 1) be replaced with the native promoter at the native gene locus of the target gene in the chromosome; or 2) be integrated with the target gene at the native gene locus of the latter or at another gene locus.

"Replacement of the native promoter at the native gene locus of the target gene" means the fact that the naturally occurring promoter of the gene which usually is naturally present by way of a single copy at its gene locus in the corresponding wild type or corresponding starting organism in the form of its nucleotide sequence is replaced.

"Another gene locus" means a gene locus whose nucleotide sequence is different from the sequence of the target gene. Said other gene locus or the nucleotide sequence at said other gene locus is preferably located within the chromosome and normally is not essential for growth and for production of the desired chemical compounds. It is furthermore possible to use intergenic regions within the chromosome, *i.e.* nucleotide sequences without coding function.

Expression or overexpression is preferably carried out in microorganisms of the genus *Corynebacterium.* Within the genus *Corynebacterium,* preference is given to strains based on the following species: *C. efficiens,* with the deposited type strain being DSM44549, *C. glutamicum,* with the deposited type strain being ATCC 13032, and *C. ammoniagenes,* with the deposited type strain being ATCC6871. Very particular preference is given to the species *C. glutamicum.*

Suitable strains of the genus Corynebacterium, in particular of the species *Corynebacterium glutamicum,* are in particular the known wild-type strains: *Corynebacterium glutamicum* ATCC 13032, *Corynebacterium acetoglutamicum* ATCC15806, *Corynebacterium acetoacidophilum* ATCC13870, *Corynebacterium melassecola* ATCC17965, *Corynebacterium thermoaminogenes* FERM BP-1539, *Brevibacterium flavum* ATCC14067, *Brevibacterium lactofermentum* ATCC13869, and *Brevibacterium divaricatum* ATCC 14020; and L-amino acid-producing mutants, or strains, prepared therefrom, such as, for example, the L-lysine-producing strains: *Corynebacterium glutamicum* FERM-P 1709, *Brevibacterium flavum* FERM-P 1708, Brevibacterium lactofermentum FERM-P 1712, *Corynebacterium glutamicum* FERM-P 6463, *Corynebacterium glutamicum* FERM-P 6464, *Corynebacterium glutamicum* DM58-1, *Corynebacterium glutamicum* DG52-5, *Corynebacterium glutamicum* DSM5714, and *Corynebacterium glutamicum DSM12866.*

The term *"Micrococcus glutamicus"* has also been in use for *C. glutamicum.* Some representatives of the species *C. efficiens* have also been referred to as *C. thermoaminogenes* in the prior art, such as the strain FERM BP-1539, for example.

The microorganisms or strains (starting strains) employed for the expression or overexpression measures according to the invention preferably already possess the ability to secrete a desired fine chemical into the surrounding nutrient medium and accumulate there. The expression "to produce" is also used for this herein below. More specifically, the strains employed for the overexpression measures possess the ability to accumulate the desired fine chemical in concentrations of at least 0.10 g/L, at least 0.25 g/L, at least 0.5 g/L, at least 1.0 g/L, at least 1.5 g/L, at least 2.0 g/L, at least 4.0 g/L, or at least 10.0 g/L in no more than 120 hours, no more than 96 hours, no more than 48 hours, no more than 36 hours, no more than 24 hours, or no more than 12 hours in the cell or in the nutrient medium. The starting strains are preferably strains prepared by mutagenesis and selection, by recombinant DNA technologies or by a combination of both methods.

A person skilled in the art understands that a microorganism suitable for the measures of the invention may also be obtained by firstly employing the promoter according to the invention of SEQ ID NO:1, SEQ ID NO:2, SEQ ID NO:3, SEQ ID NO:4, SEQ ID NO:5, SEQ ID NO:6, SEQ ID NO:7, or SEQ ID NO:8 for overexpression of the target genes in a wild strain such as, for example, the *C. glutamicum* type strain ATCC 13032 or the strain ATCC 14067, and then, by means of further genetic measures described in the prior art, causing the microorganism to produce the desired fine chemical(s).

The term "biomolecules" means with regard to the measures of the invention amino acids, organic acids, vitamins, nucleosides and nucleotides. Particular preference is given to proteinogenic amino acids, non-proteinogenic amino acids, macromolecules, and organic acids.

"Proteinogenic amino acids" mean the amino acids which occur in natural proteins, *i*.*e.* in proteins of microorganisms, plants, animals and humans. They serve as structural units for proteins in which they are linked to one another via peptide bonds.

Where L-amino acids or amino acids are mentioned hereinbelow, they are to be understood as meaning one or more amino acids, including their salts, selected from the group L-asparagine, L-threonine, L-serine, L-glutamate, L-glycine, L-alanine, L-cysteine, L-valine, L-methionine, L-isoleucine, L-leucine, L-tyrosine, L-phenylalanine, L-histidine, L-lysine, L-tryptophan and L-arginine. L-lysine is especially preferred. L-Amino acids, in particular lysine, are used in human medicine and in the pharmaceuticals industry, in the foodstuffs industry and very particularly in animal nutrition. There is therefore a general interest in providing new improved processes for the preparation of amino acids, in particular L-lysine.

The terms protein and polypeptide are interchangeable.

The present invention provides a microorganism which produces a fine chemical, said microorganism having increased expression of one or more genes in comparison to the particular starting strain by using the promoter according to the invention of SEQ ID NO:1, SEQ ID NO:2, SEQ ID NO:3, SEQ ID NO:4, SEQ ID NO:5, SEQ ID NO:6, SEQ ID NO:7, or SEQ ID NO:8.

### Fermentative Preparation

The present invention furthermore provides a process for fermentative preparation of a fine chemical, comprising the steps of:
a) culturing the above-described microorganism according to the present invention in a suitable medium, resulting in a fermentation broth; and
b) concentrating the fine chemical in the fermentation broth of a) and/or in the cells of the microorganism.

Preference is given here to obtaining from the fine chemical-containing fermentation broth the fine chemical or a liquid or solid fine chemical-containing product. The microorganisms produced may be cultured continuously-as described, for example, in WO 05/021772-or discontinuously in a batch process (batch cultivation) or in a fed-batch or repeated fed-batch process for the purpose of producing the desired organic-chemical compound. A summary of a general nature about known cultivation methods is available in the textbook by Chmiel (Bioprozeßtechnik. 1: Einführung in die Bioverfahrenstechnik (Gustav Fischer Verlag, Stuttgart, 1991)) or in the textbook by Storhas (Bioreaktoren and periphere Einrichtungen (Vieweg Verlag, Braunschweig/Wiesbaden, 1994)).

The culture medium or fermentation medium to be used must in a suitable manner satisfy the demands of the respective strains. Descriptions of culture media for various microorganisms are present in the "Manual of Methods for General Bacteriology" of the American Society for Bacteriology (Washington D.C., USA, 1981). The terms culture medium and fermentation medium are interchangeable.

It is possible to use, as carbon source, sugars and carbohydrates such as, for example, glucose, sucrose, lactose, fructose, maltose, molasses, sucrose-containing solutions from sugar beet or sugar cane processing, starch, starch hydrolysate, and cellulose; oils and fats such as, for example, soybean oil, sunflower oil, groundnut oil and coconut fat; fatty acids such as, for example, palmitic acid, stearic acid, and linoleic acid; alcohols such as, for example, glycerol, methanol, and ethanol; and organic acids such as, for example, acetic acid or lactic acid.

It is possible to use, as nitrogen source, organic nitrogen-containing compounds such as peptones, yeast extract, meat extract, malt extract, corn steep liquor, soybean flour, and urea; or inorganic compounds such as ammonium sulfate, ammonium chloride, ammonium phosphate, ammonium carbonate, and ammonium nitrate. The nitrogen sources can be used individually or as a mixture.

It is possible to use, as phosphorus source, phosphoric acid, potassium dihydrogen phosphate or dipotassium hydrogen phosphate or the corresponding sodium-containing salts.

The culture medium may additionally comprise salts, for example in the form of chlorides or sulfates of metals such as, for example, sodium, potassium, magnesium, calcium and iron, such as, for example, magnesium sulfate or iron sulfate, which are necessary for growth. Finally, essential growth factors such as amino acids, for example homoserine and vitamins, for example thiamine, biotin or pantothenic acid, may be employed in addition to the abovementioned substances.

Said starting materials may be added to the culture in the form of a single batch or be fed in during the cultivation in a suitable manner.

The pH of the culture can be controlled by employing basic compounds such as sodium hydroxide, potassium hydroxide, ammonia, or aqueous ammonia; or acidic compounds such as phosphoric acid or sulfuric acid in a suitable manner. The pH is generally adjusted to a value of from 6.0 to 8.5, preferably 6.5 to 8. To control foaming, it is possible to employ antifoams such as, for example, fatty acid polyglycol esters. To maintain the stability of plasmids, it is possible to add to the medium suitable selective substances such as, for example, antibiotics. The fermentation is preferably carried out under aerobic conditions. In order to maintain these conditions, oxygen or oxygen-containing gas mixtures such as, for example, air are introduced into the culture. It is likewise possible to use liquids enriched with hydrogen peroxide. The fermentation is carried out, where appropriate, at elevated pressure, for example at an elevated pressure of from 0.03 to 0.2 MPa. The temperature of the culture is normally from 20 °C to 45 °C and preferably from 25 °C to 40 °C, particularly preferably from 30 °C to 37 °C. In batch or fed-batch processes, the cultivation is preferably continued until an amount of the desired organic-chemical compound sufficient for being recovered has formed. This aim is normally achieved within 10 hours to 160 hours. In continuous processes, longer cultivation times are possible. The activity of the microorganisms results in a concentration (accumulation) of the organic-chemical compound in the fermentation medium and/or in the cells of said microorganisms.

Examples of suitable fermentation media can be found *inter alia* in the patents US 5,770,409, US 5,990,350, US 5,275,940, WO 2007/012078, US 5,827,698, WO 2009/043803, US 5,756,345 and US 7,138,266.

Analysis of L-amino acids to determine the concentration at one or more time(s) during the fermentation can take place by separating the L-amino acids by means of ion exchange chromatography, preferably cation exchange chromatography, with subsequent post-column derivatization using ninhydrin, as described in Spackman et al. (Analytical Chemistry 30:1190-1206 (1958)). It is also possible to employ *ortho*-phthaldialdehyde rather than ninhydrin for post-column derivatization. An overview article on ion exchange chromatography can be found in Pickering (LC-GC Magazine of Chromatographic Science) 7(6), 484-487 (1989)).

It is likewise possible to carry out a pre-column derivatization, for example using *ortho-*phthaldialdehyde or phenyl isothiocyanate, and to fractionate the resulting amino acid derivates by reversed-phase (RP) chromatography, preferably in the form of high-performance liquid chromatography (HPLC). A method of this type is described, for example, in Lindroth et al. (Analytical Chemistry 51:1167-1174 (1979)).

Detection is carried out photometrically (absorption, fluorescence).

A review regarding amino acid analysis can be found *inter alia* in the textbook "Bioanalytik" from Lottspeich and Zorbas (Spektrum Akademischer Verlag, Heidelberg, Germany 1998).

Determination of the concentration of α-ketoacids at one or more time point(s) in the course of the fermentation may be carried out by separating the ketoacids and other secreted products by means of ion exchange chromatography, preferably cation exchange chromatography, on a sulfonated styrenedivinylbenzene polymer in the H+ form, for example by means of 0.025 M sulfuric acid with subsequent UV detection at 215 nm (alternatively also at 230 or 275 nm). Preferably, a REZEK RFQ - Fast Fruit H+ column (Phenomenex) may be employed, but other suppliers for the separating phase (*e.g.* Aminex from BioRad) are feasible. Similar separations are described in application examples by the suppliers.

The performance of the processes or fermentation processes containing the promoter variants according to the invention, in terms of one or more of the parameters selected from the group of concentration (compound formed per unit volume), yield (compound formed per unit carbon source consumed), formation (compound formed per unit volume and time) and specific formation (compound formed per unit dry cell matter or dry biomass and time or compound formed per unit cellular protein and time) or else other process parameters and combinations thereof, is increased by at least 0.5%, at least 1%, at least 1.5%, at least 2%, at least 3%, at least 4%, at least 5%, at least 10%, at least 20%, at least 30%, at least 40%, at least 50%, at least 60%, at least 70%, at least 80%, at least 90%, or at least 100% based on processes or fermentation processes using microorganisms not containing the promoter variants according to the invention. This is considered to be very worthwhile in terms of a large-scale industrial process.

The fermentation measures result in a fermentation broth which contains the desired fine chemical, preferably amino acids, organic acids, vitamins, nucleosides or nucleotides.

A product containing the fine chemical is then provided or produced or recovered in liquid or solid form.

A fermentation broth means a fermentation medium or nutrient medium in which a microorganism has been cultivated for a certain time and at a certain temperature. The fermentation medium or the media employed during fermentation comprise(s) all the substances or components which ensure production of the desired compound and typically propagation and viability.

When the fermentation is complete, the resulting fermentation broth accordingly comprises:
a) the biomass (cell mass) of the microorganism, said biomass having been produced due to propagation of the cells of said microorganism;
b) the desired fine chemical formed during the fermentation;
c) the organic byproducts possibly formed during the fermentation; and
d) the constituents of the fermentation medium employed or of the starting materials, such as, for example, vitamins such as biotin or salts such as magnesium sulfate, which have not been consumed in the fermentation.

The organic byproducts include substances which are produced by the microorganisms employed in the fermentation in addition to the particular desired compound and are optionally secreted.

The fermentation broth is removed from the culture vessel or fermentation tank, collected where appropriate, and used for providing a product containing the fine chemical in liquid or solid form. The expression "recovering the fine chemical-containing product" is also used for this. In the simplest case, the fine chemical-containing fermentation broth itself, which has been removed from the fermentation tank, constitutes the recovered product.

One or more of the measures selected from the group consisting of
a) partial (> 0% to < 80%) to complete (100%) or virtually complete (≥ 80%, ≥ 90%, ≥ 95%, ≥ 96%, ≥ 97%, ≥ 98%, or ≥ 99%) removal of the water;
b) partial (> 0% to < 80%) to complete (100%) or virtually complete (≥ 80%, ≥ 90%, ≥ 95%, ≥ 96%, ≥ 97%, ≥ 98%, or ≥ 99%) removal of the biomass, the latter being optionally inactivated before removal;
c) partial (> 0% to < 80%) to complete (100%) or virtually complete (≥ 80%, ≥ 90%, ≥ 95%, ≥ 96%, ≥ 97%, ≥ 98%, ≥ 99%, ≥ 99.3%, or ≥ 99.7%) removal of the organic byproducts formed during fermentation; and
d) partial (> 0%) to complete (100%) or virtually complete (≥ 80%, ≥ 90%, ≥ 95%, ≥ 96%, ≥ 97%, ≥ 98%, ≥ 99%, ≥ 99.3%, or ≥ 99.7%) removal of the constituents of the fermentation medium employed or of the starting materials, which have not been consumed in the fermentation, from the fermentation broth achieves concentration or purification of the desired organic-chemical compound. Products having a desired content of said compound are isolated in this way.

The partial (> 0% to < 80%) to complete (100%) or virtually complete (≥ 80% to < 100%) removal of the water (measure a)) is also referred to as drying.

In one variant of the process, complete or virtually complete removal of the water, of the biomass, of the organic byproducts and of the unconsumed constituents of the fermentation medium employed results in pure (≥ 80% by weight, ≥ 90% by weight) or high-purity (≥ 95% by weight, ≥ 97% by weight, or ≥ 99% by weight) product forms of the desired organic-chemical compound. An abundance of technical instructions for measures a), b), c) and d) are available in the prior art.

Depending on requirements, the biomass can be removed wholly or partly from the fermentation broth by separation methods such as, for example, centrifugation, filtration, decantation or a combination thereof, or be left completely therein. Where appropriate, the biomass or the biomass-containing fermentation broth is inactivated during a suitable process step, for example by thermal treatment (heating) or by addition of acid.

In one procedure, the biomass is completely or virtually completely removed so that no (0%) or at most 30%, at most 20%, at most 10%, at most 5%, at most 1% or at most 0.1% biomass remains in the prepared product. In a further procedure, the biomass is not removed, or is removed only in small proportions, so that all (100%) or more than 70%, 80%, 90%, 95%, 99% or 99.9% biomass remains in the product prepared. In one process according to the invention, accordingly, the biomass is removed in proportions of from ≥ 0% to ≤ 100%.

Finally, the fermentation broth obtained after the fermentation can be adjusted, before or after the complete or partial removal of the biomass, to an acidic pH with an inorganic acid such as, for example, hydrochloric acid, sulfuric acid, or phosphoric acid; or organic acid such as, for example, propionic acid, so as to improve the handling properties of the final product (GB 1,439,728 or EP 1 331220). It is likewise possible to acidify the fermentation broth with the complete content of biomass. Finally, the broth can also be stabilized by adding sodium bisulfite (NaHCO₃, GB 1,439,728) or another salt, for example ammonium, alkali metal, or alkaline earth metal salt of sulfurous acid.

During the removal of the biomass, any organic or inorganic solids present in the fermentation broth are partially or completely removed. The organic byproducts dissolved in the fermentation broth, and the dissolved unconsumed constituents of the fermentation medium (starting materials), remain at least partly (> 0%), preferably to an extent of at least 25%, particularly preferably to an extent of at least 50% and very particularly preferably to an extent of at least 75% in the product. Where appropriate, they also remain completely (100%) or virtually completely, meaning > 95% or > 98% or > 99%, in the product. If a product in this sense comprises at least part of the constituents of the fermentation broth, this is also described by the term "product based on fermentation broth".

Subsequently, water is removed from the broth, or said broth is thickened or concentrated, by known methods such as, for example, using a rotary evaporator, thin-film evaporator, falling-film evaporator, by reverse osmosis or by nanofiltration. This concentrated fermentation broth can then be worked up to free-flowing products, in particular to a fine powder or preferably coarse granules, by methods of freeze drying, spray drying, spray granulation or by other processes such as in the circulating fluidized bed, as described for example according to PCT/EP2004/006655. A desired product is isolated where appropriate from the resulting granules by screening or dust removal. It is likewise possible to dry the fermentation broth directly, *i.e.* without previous concentration by spray drying or spray granulation.

"Free-flowing" means powders which, from a series of glass orifice vessels with orifices of different sizes, flow unimpeded at least out of the vessel with a 5 mm orifice (Klein: Seifen, Ole, Fette, Wachse 94, 12 (1968)).

"Fine" means a powder predominantly (> 50%) having a particle size of diameter from 20 to 200 µm.

"Coarse" means a product predominantly (> 50%) of a particle size of diameter from 200 to 2000 µm.

The particle size determination can be carried out by methods of laser diffraction spectrometry. Corresponding methods are described in the textbook "Teilchengrößenmessung in der Laborpraxis" by R. H. Müller and R. Schuhmann, Wissenschaftliche Verlagsgesellschaft Stuttgart (1996) or in the text book "Introduction to Particle Technology" by M. Rhodes, published by Wiley &Sons (1998).

The free-flowing, fine powder can in turn be converted by suitable compaction or granulation processes into a coarse, very free-flowing, storable and substantially dust-free product.

The term "dust-free" means that the product comprises only small proportions (< 5%) of particle sizes below 100 µm in diameter.

"Storable" in the sense of this invention means a product which can be stored for at least one (1) year or longer, preferably at least 1.5 years or longer, particularly preferably two (2) years or longer, in a dry and cool environment without any substantial loss of the respective organic-chemical compound occurring. "Substantial loss" means a loss of >5%.

It is advantageous to employ during the granulation or compaction the usual organic or inorganic auxiliaries or carriers such as starch, gelatin, cellulose derivatives or similar substances, as normally used in the processing of food products or feeds as binders, gelling agents or thickeners, or further substances such as, for example, silicas, silicates (EP0743016A) and stearates.

It is further advantageous to treat the surface of the resulting granules with oils or fats as described in WO04/054381. Oils which can be used are mineral oils, vegetable oils or mixtures of vegetable oils. Examples of such oils are soybean oil, olive oil, soybean oil/lecithin mixtures. In the same way, silicone oils, polyethylene glycols or hydroxyethylcellulose are also suitable. Treatment of the surfaces of the granules with said oils achieves an increased abrasion resistance of the product and a reduction in the dust content. The oil content in the product is 0.02 to 2.0% by weight, preferably 0.02 to 1.0% by weight, and very particularly preferably 0.2 to 1.0% by weight, based on the total amount of the feed additive.

Preferred products have a proportion of ≥ 97% by weight with a particle size of from 100 to 1800 µm or a proportion of ≥ 95% by weight with a particle size of diameter 300 to 1800 µm. The proportion of dust, *i.e.* particles with a particle size < 100 pm, is preferably > 0 to 1% by weight, particularly preferably not exceeding 0.5% by weight.

However, alternatively, the product may also be absorbed on an organic or inorganic carrier known and customary in the processing of feeds, such as, for example, silicas, silicates, meals, brans, flours, starches, sugars or others, and/or be mixed and stabilized with customary thickeners or binders. Examples of use and processes therefor are described in the literature (Die Mühle + Mischfuttertechnik 132 (1995) 49, page 817).

### EXAMPLES

### Example 1: Identification of Candidate Promoters.

The following procedure was used to identify native *C. glutamicum* promoters that satisfied both of the following criteria: 1) represented a ladder of constitutive promoters; and 2) could be encoded by short DNA sequences, ideally less than 100 base pairs. A published data set describing global gene expression levels in *C. glutamicum* ATCC 13032 (Lee et al., Biotechnology Letters, 2013) was examined to identify genes that were constitutively expressed across different growth conditions. Genes whose expression level remained constant (defined as a ratio of expression between 0.33 and 3) across two growth conditions, namely chemostat growth in minimal media with and without the addition of hydrogen peroxide satisfied the first criterion. A published data set describing the *C. glutamicum* ATCC 13032 transcriptome (Pfeifer-Sancar et al., BMC Genomics 2013, 14:888) was examined to find genes with compact promoters, *i.e.* those consisting of a 60 base pair core promoter region and a 5 prime untranslated region between 26 and 40 base pairs in length. The two data sets were cross-referenced to identify promoters that satisfied both criteria. See Figure 1. Five candidate promoters (SEQ ID NOs: 2, 3, 4, 6, and 8) were selected for further evaluation.

### Example 2: Evaluation of Candidate Promoter Activity

To evaluate candidate promoter activity, a set of plasmid based fluorescence reporter constructs was designed. Briefly, each promoter was cloned in front of *eyfp,* a gene encoding yellow fluorescent protein in the shuttle vector pK18rep. These plasmids were transformed into *C. glutamicum* NRRL B-11474 and promoter activity was assessed by measuring the accumulation of YFP protein by spectrometry.

The shuttle vector pK18rep was constructed by replacing the sacB gene in pK18mobSacB (ATCC 87087) with the pBL1 origin of replication (GenBank: AF092037.1) resulting in a vector able to propagate in both *E. coli* and *C. glutamicum.* Briefly, we PCR amplified a portion of pK18mobSacB containing the *E. coli* origin of replication and the Kanamycin resistance gene nptII using the primers pK18F (TCATGACCAAAATCCCTTAACGTG (SEQ ID NO:9)) and pK18R (GCGTACTCTTCGATGGTGAAAACATCTC (SEQ ID NO:10)) and PCR amplified synthetic DNA encoding the pBLl origin of replication with the primers pBLIF (GACCTAAAATGTGTAAAGGGCAAAGTGTATACaacaacaagacccatcatagtttgc (SEQ ID NO:11)) and pBL1R (CACGTTAAGGGATTTTGGTCATGAcacatgcagtcatgtcgtgc (SEQ ID NO:12)). The PCR products were treated with DpnI (New England Biolabs) when appropriate, purified with DNA Clean & Concentrate-5 (Zymo Research), and assembled using the Gibson Assembly method with Gibson Assembly Master Mix (NEB) according to manufactures instructions. The Gibson Assembly reaction was transformed into NEB Turbo competent cells (New England Biolabs) according to the manufactures instructions. Transformants were selected on LB agar plus 25 µg/mL Kanamycin and verified by Sanger sequencing.

The reporter construct pK18rep-Psod-eyfp was constructed by restriction digestion and ligation of pK18rep and a synthetic DNA construct consisting of 191 base pair DNA sequence that encodes the superoxide disumutase (GenBank: BA000036.3) promoter from *C. glutamicum* ATCC 13032 upstream of eyfp gene followed by a 77 base pair DNA sequence encoding the sod terminator from *C. glutamicum* ATCC 13032 flanked by EcoRI and Sall restriction sites. The parent vector and synthetic DNA insert were digested with EcoRI-HF and SalI-HF (New England Biolabs) and the resulting products were run on an agarose gel. The DNA was extracted from the gel and purified using the Zymoclean Gel DNA Recovery Kit (Zymo Research) and ligated with T4 DNA ligase (New England Biolabs) according to the manufactures instructions. The ligation reaction was transformed into NEB Turbo competent cells (New England Biolabs) according to the manufactures instructions. Transformants were selected on LB agar plus 25 µg/mL Kanamycin and verified by Sanger sequencing.

Additional promoter reporter constructs were constructed by replacing the sod promoter in pK18rep-Psod-eyfp. We PCR amplified pK18rep-Psod-eyfp excluding the sod promoter with primers pK18repR (gcttgcatgcctgcaggtcga (SEQ ID NO: 13)) and yfpF (ATGGTGAGCAAGGGCGAGGAGC (SEQ ID NO: 14)). The PCR product was treated with DpnI (New England Biolabs) and purified with DNA Clean & Concentrate-5 (Zymo Research) and assembled with synthetic DNA constructs encoding the promoter of interest plus 25 base pair homology sequence to the destination vector using the Gibson Assembly method with Gibson Assembly Master Mix (NEB) according to manufactures instructions. The Gibson Assembly reaction was transformed into NEB Turbo competent cells (New England Biolabs) according to the manufacturer's instructions. Transformants were selected on LB agar plus 25 µg/mL Kanamycin and verified by Sanger sequencing.

Additionally, the strong constitutive promoter Pcg0007 (SEQ ID NO:2) was chosen for mutagenesis. In *C. glutamicum,* the -10 element is thought to play a key role in determining promoter activity (Pfeifer-Sancar et al., BMC Genomics 2013, 14:888) therefore four out of six positions in predicted -10 element (TAAGAT) of Pc0007 were randomized in order to generate both stronger and attenuated promoter variants (SEQ ID NOs 1, 5, and 7). This library was generated by PCR amplifying pK18rep-Pc0007-eyfp with Pcg0007Fwd (GGAAACGTCTGTATCGGATAAGTAG (SEQ ID NO: 15)) and Pc0007Rev (CTACTTATCCGATACAGACGTTTCCANNNNACACGCTTAGGTCCCCACGTAGTACCA (SEQ ID NO: 16)), treated with DpnI (New England Biolabs) and assembled using the Gibson Assembly method with Gibson Assembly Master Mix (NEB) according to manufactures instructions. The Gibson Assembly reaction was transformed into NEB Turbo competent cells (New England Biolabs) according to the manufacturer's instructions. Transformants were selected on LB agar plus 25 µg/mL Kanamycin and individual colonies were characterized by Sanger sequencing. Colonies were pooled by scraping the agar and purified plasmid DNA was isolated using the Zyppy minipred kit (Zymo Research).

Purified reporter construct plasmids were transformed into *C. glutamicum* NRRL B-11474 by electroporation (Haynes et al., Journal of General Microbiology, 1990). Transformants were selected on BHI agar plus 25 µg/mL Kanamycin. For each transformation, multiple single colonies were picked and inoculated into individual wells of a 96 mid-well block containing 300 µL of BHI media plus 25 µg/mL Kanamycin. The cells were grown to saturation by incubation for 48 h at 30 °C shaking at 1,000 rpm. After incubation, cultures were centrifuged for 5 min at 3,500 rpm and the media was removed by aspiration. Cells were washed once by resuspension in 300 µL of PBS and centrifugation for 5 min at 3,500 rpm followed by aspiration of the supernatant and a final resuspension in 300 µL of PBS. A 20 µL aliquot of this mixture was transferred to a 96-well full area black clear bottom assay plate containing 180 µL of PBS. The optical density of the cells at 600 nm was measured with the SpectraMax M5 microplate reader and the fluorescence was measured with the TECAN M1000 microplate leader by exciting at 514 nm and measuring emission at 527 nm. For each well a normalized fluorescence activity was calculated by dividing fluorescence by optical density. The parent plasmid pK18rep acted as a negative cantrol. Normalized fluorescence activity was compared between reporter constructs and between biological replicates (Figure 2). A numerical summary of promoter activity is presented in Table 5 below.

**Table 5: Recombinant C. glutamicum Expressing Yellow Fluorescent Protein Under the Control of Promoters**

| **Strain** | **SEQ ID NO** | **No. of Replicates** | **Mean Activity** | **Standard Deviation** | **Standard Error of Mean** | **95% Confidence Interval** | **Relative Expression** |
|---|---|---|---|---|---|---|---|
| 0007_lib_39 | 1 | 12 | 114402 | 52987.9 | 15296 | 80735-148069 | 1167 |
| Pcg1860-eyfp | 2 | 19 | 89243 | 16162.2 | 3708 | 81453-97033 | 911 |
| Pcg0007-eyfp | 3 | 19 | 44527 | 18110.3 | 4155 | 35798-53256 | 454 |
| Pcg0755-eyfp | 4 | 10 | 43592 | 3643 | 1152 | 40986-46198 | 445 |
| 0007_lib_265 | 5 | 11 | 11286 | 10459.4 | 3154 | 4260-18313 | 115 |
| Pcg3381-eyfp | 6 | 19 | 4723 | 1854.3 | 425 | 3829-5617 | 48 |
| 0007_lib_119 | 7 | 18 | 661 | 731.9 | 173 | 297-1025 | 7 |
| Pcg3121-eyfp | 8 | 14 | 98 | 537.5 | 144 | -212-409 | 1 |
| pK18rep | - | 20 | -45 | 214.9 | 48 | -145-56 | |

### Example 3: Application of Candidate Promoters to the L-lysine Biosynthetic Pathway

The promoters of the present disclosure are useful for improved processes for the production of biomolecules in host cells. An example of the application and use of the promotor of the present disclosure is directed to the production of the amino acid L-lysine. Figure 3 presents the biosynthetic pathway for the production of L-lysine and includes the genes *pck, odx, icd,* and *hom (e,g.,* the homoserine/threonine synthase pathway), that divert intermediates from the pathway leading to reductions in overall L-lysine yield. The symbols, gene names, Enzyme Commission number (EC number), and map position in *C. glutamicum* strain ATCC 13032 are provided below in Table 3.

Recombinant vectors comprising a promoter of SEQ ID NOs: 1 to 8 functionally linked to a target gene as provided in Table 3 are cloned into *Corynebacterium* cloning vectors using yeast homologous recombination cloning techniques to assemble a vector in which each promoter was flanked by direct repeat regions to provide for homologous recombination in *Corynebacterium glutamican* at the target gene locus. Upon recombination, the endogenous promoter is replaced by the promoter of SEQ ID NOs: 1 to 8 functionally linked to the respective target gene in the endogenous *C. glutamican* locus. A variety of targeting vectors comprising the promoter and functionally linked target gene included a range of homology direct repeat arm lengths ranging from 0.5Kb, 1Kb, 2Kb, and 5Kb. Each DNA insert was produced by PCR amplification of homologous regions using commercially sourced oligos and the host strain genomic DNA described above as template. The promoter to be introduced into the genome was encoded in the oligo tails. PCR fragments were assembled into the vector backbone using homologous recombination in yeast.

Vectors are initially transformed into *E.coli* using standard heat shock transformation techniques and correctly assembled clones are identified and validated. Transformed *E.coli* bacteria are tested for assembly success. Four colonies from each *E*. *coli* transformation plate are cultured and tested for correct assembly via PCR. Vectors are amplified in the *E. coli* hosts to provide vector DNA for Corynebacterium transformation.

Validated clones are transformed into *Corynebacterium glutamicum* host cells via electroporation. For each transformation, the number of Colony Forming Units (CFUs) per µg of DNA is determined as a function of the insert size. Coryne genome integration is analyzed as a function of homology arm length. Shorter arms had a lower efficiency.

Cultures of *Corynebacterium* identified as having successful integrations of the insert cassette are cultured on media containing 5% sucrose to counter select for loop outs of the *sacb* selection gene. Sucrose resistance frequency for various homology direct repeat arms do not vary significantly with arm length. These results suggest that loopout efficiencies remain steady across homology arm lengths of 0.5 kb to 5kb.

In order to further validate loop out events, colonies exhibiting sucrose resistance are cultured and analyzed via sequencing. The results for the sequencing of the insert genomic regions are summarized below in Table 6.

**Table 6: Loop-out Validation Frequency**

| **Outcome** | **Frequency (sampling error 95% confidence)** |
|---|---|
| **Successful Loop out** | 13% (9%/20%) |
| **Loop Still present** | 42% (34%/50%) |
| **Mixed read** | 44% (36%/52%) |

Sequencing results show a 10-20% efficiency in loop outs. Not to be limited by any particular theory, loop-out may be dependent on insert sequence. Even if correct, picking 10-20 sucrose-resistant colonies leads to high success rates.

Upon integration, the recombinant vectors replace the endogenous promoter sequences with a promoter selected from the group consisting of Pcg1860 (SEQ ID NO:2), Pcg0007 (SEQ ID NO:3), Pcg0755 (SEQ ID NO:4), Pcg0007_lib_265 (SEQ ID NO:5), Pcg3381 (SEQ ID NO:6), Pcg007_lib_119 (SEQ ID NO:7), and Pcg3121 (SEQ ID NO:8). A list of the resulting recombinant strains is provided below in Table 7.

Multiple single colonies (N in Table 7) are picked, inoculated and grown as a small scale culture. Each newly created strain comprising a test promoter is tested for lysine yield in small scale cultures designed to assess product titer performance. Small scale cultures are conducted using media from industrial scale cultures. Product titer is optically measured at carbon exhaustion (*i.e*., representative of single batch yield) with a standard colorimetric assay. Briefly, a concentrated assay mixture is prepared and is added to fermentation samples such that final concentrations of reagents are 160 mM sodium phosphate buffer, 0.2 mM Amplex Red, 0.2 U/mL Horseradish Peroxidase and 0.005 U/mL of lysine oxidase. Reactions proceed to completion and optical density is measured using a Tecan M1000 plate spectrophotometer at a 560nm wavelength.

As shown in Table 7, the yield of L-lysine is increased by over 24% (*e.g.,* recombinant strain 7000007840) over the non-engineered strain. In other embodiments, the yield of L-lysine is decreased by nearly 90% (e.g., recombinant strain 700000773). As provided in Table 7, replacement of the promoter for the pgi and zwf results in greater than 10% improvements to L-lysine production.

Notably, the production of L-lysine is not a simple dependence on incorporating the most active promoters. As illustrated in Figure 4, lysine yield is maximized by a relatively weak promoter (*e.g.,* pgi having relative promoter expression of 1, 7x, or 48x, or dapB at a relative promoter strength of 7x) or maximized by intermediate expression (*e.g.,* lysA at having a relative promoter expression of 454x). In certain cases, expression is maximal when the relative promoter strength is maximized (e.g., ppc), As exemplified Figure 5, the location of the gene in the genetic pathway (Figure 3) does not reliably predict the relative increase or decrease in L-lysine yield or the optimal promoter strength. For example, high level expression of cg0931 results in improved yield while higher levels of dapD result in no improvement or decreased yield.

**Table 7: Recombinant strains of C. glutamicum having modified expression of L-lysine Biosynthetic Genes**

| **Strain** | **promoter-target** | **N** | **Mean (A₅₆₀)** | **Std Error** | **% Yield Change From Base** |
|---|---|---|---|---|---|
| 7000007713 | Pcg1860-asd | 8 | 0.84595 | 0.00689 | 3.927615 |
| 7000007736 | Pcg0755-asd | 4 | 0.84036 | 0.00974 | 3.240866 |
| 7000007805 | Pcg0007_119-asd | 8 | 0.82493 | 0.00689 | 1.345242 |
| 7000007828 | Pcg3121-asd | 8 | 0.8246 | 0.00689 | 1.3047 |
| 7000007759 | Pcg0007_265-asd | 8 | 0.81155 | 0.00689 | -0.29853 |
| 7000007782 | Pcg3381-asd | 8 | 0.8102 | 0.00689 | -0.46438 |
| 7000007712 | Pcg1860-ask | 8 | 0.83958 | 0.00689 | 3.14504 |
| 7000007735 | Pcg0755-ask | 8 | 0.81673 | 0.00689 | 0.337846 |
| 7000007827 | Pcg3121-ask | 8 | 0.81498 | 0.00689 | 0.122853 |
| 7000007804 | Pcg0007_119-ask | 8 | 0.81492 | 0.00689 | 0.115482 |
| 7000007758 | Pcg0007_265-ask | 8 | 0.80381 | 0.00689 | -1.24942 |
| 7000007781 | Pcg3381-ask | 8 | 0.80343 | 0.00689 | -1.2961 |
| 7000007780 | Pcg3381-aspB | 8 | 0.84072 | 0.00689 | 3.285093 |
| 7000007803 | Pcg0007_119-aspB | 8 | 0.82106 | 0.00689 | 0.8698 |
| 7000007809 | Pcg0007_119-cg0931 | 8 | 0.83446 | 0.00689 | 2.516032 |
| 7000007717 | Pcg1860-cg0931 | 4 | 0.83129 | 0.00974 | 2.126588 |
| 7000007763 | Pcg0007_265-cg0931 | 4 | 0.82628 | 0.00974 | 1.511094 |
| 7000007671 | Pcg0007_39-cg0931 | 8 | 0.82554 | 0.00689 | 1.420182 |
| 7000007740 | Pcg0755-cg0931 | 8 | 0.81921 | 0.00689 | 0.642522 |
| 7000007694 | Pcg0007-cg0931 | 8 | 0.80444 | 0.00689 | -1.17202 |
| 7000007691 | Pcg0007-dapA | 8 | 0.8299 | 0.00689 | 1.955822 |
| 7000007783 | Pcg3381-dapA | 8 | 0.80951 | 0.00689 | -0.54915 |
| 7000007760 | Pcg0007_265-dapA | 8 | 0.76147 | 0.00689 | -6.45102 |
| 7000007806 | Pcg0007_119-dapA | 8 | 0.35394 | 0.00689 | -56.5174 |
| 7000007761 | Pcg0007_265-dapB | 8 | 0.84157 | 0.00689 | 3.389518 |
| 7000007738 | Pcg0755-dapB | 4 | 0.84082 | 0.00974 | 3.297378 |
| 7000007692 | Pcg0007-dapB | 8 | 0.83088 | 0.00689 | 2.076218 |
| 7000007784 | Pcg3381-dapB | 8 | 0.82474 | 0.00689 | 1.3219 |
| 7000007715 | Pcg1860-dapB | 8 | 0.82232 | 0.00689 | 1.024595 |
| 7000007830 | Pcg3121-dapB | 8 | 0.81236 | 0.00689 | -0.19902 |
| 7000007807 | Pcg0007_119-dapB | 4 | 0.69622 | 0.00974 | -14.4672 |
| 7000007762 | Pcg0007_265-dapD | 8 | 0.84468 | 0.00689 | 3.771591 |
| 7000007808 | Pcg0007_119-dapD | 8 | 0.83869 | 0.00689 | 3.035701 |
| 7000007785 | Pcg3381-dapD | 8 | 0.83397 | 0.00689 | 2.455834 |
| 7000007670 | Pcg0007_39-dapD | 8 | 0.81698 | 0.00689 | 0.368559 |
| 7000007831 | Pcg3121-dapD | 4 | 0.8155 | 0.00974 | 0.186737 |
| 7000007693 | Pcg0007-dapD | 8 | 0.8117 | 0.00689 | -0.28011 |
| 7000007716 | Pcg1860-dapD | 8 | 0.79044 | 0.00689 | -2.89196 |
| 7000007739 | Pcg0755-dapD | 8 | 0.78694 | 0.00689 | -3.32195 |
| 7000007787 | Pcg3381-dapE | 8 | 0.83814 | 0.00689 | 2.968132 |
| 7000007833 | Pcg3121-dapE | 8 | 0.83721 | 0.00689 | 2.853878 |
| 7000007741 | Pcg0755-dapE | 8 | 0.83263 | 0.00689 | 2.291211 |
| 7000007810 | Pcg0007_119-dapE | 8 | 0.83169 | 0.00689 | 2.175729 |
| 7000007718 | Pcg1860-dapE | 8 | 0.81855 | 0.00689 | 0.561439 |
| 7000007672 | Pcg0007_39-dapE | 8 | 0.80932 | 0.00689 | -0.5725 |
| 7000007765 | Pcg0007_265-dapF | 8 | 0.8327 | 0.00689 | 2.299811 |
| 7000007788 | Pcg3381-dapF | 8 | 0.82942 | 0.00689 | 1.896853 |
| 7000007811 | Pcg0007_119-dapF | 8 | 0.82926 | 0.00689 | 1.877196 |
| 7000007696 | Pcg0007-dapF | 8 | 0.82099 | 0.00689 | 0.861201 |
| 7000007719 | Pcg1860-dapF | 8 | 0.82067 | 0.00689 | 0.821888 |
| 7000007673 | Pcg0007_39-dapF | 8 | 0.82062 | 0.00689 | 0.815745 |
| 7000007789 | Pcg3381-ddh | 8 | 0.84817 | 0.00689 | 4.200349 |
| 7000007835 | Pcg3121-ddh | 8 | 0.82141 | 0.00689 | 0.912799 |
| 7000007812 | Pcg0007_119-ddh | 8 | 0.82093 | 0.00689 | 0.853829 |
| 7000007674 | Pcg0007_39-ddh | 8 | 0.81494 | 0.00689 | 0.117939 |
| 7000007720 | Pcg1860-ddh | 8 | 0.81473 | 0.00689 | 0.09214 |
| 7000007766 | Pcg0007_265-ddh | 8 | 0.81427 | 0.00689 | 0.035627 |
| 7000007743 | Pcg0755-ddh | 8 | 0.80655 | 0.00689 | -0.9128 |
| 7000007697 | Pcg0007-ddh | 8 | 0.80621 | 0.00689 | -0.95457 |
| 7000007779 | Pcg3381-fbp | 8 | 0.85321 | 0.00689 | 4.819529 |
| 7000007802 | Pcg0007_119-fbp | 4 | 0.81425 | 0.00974 | 0.03317 |
| 7000007710 | Pcg1860-fbp | 4 | 0.40253 | 0.00974 | -50.5479 |
| 7000007687 | Pcg0007-fbp | 8 | 0.14881 | 0.00689 | -81.7182 |
| 7000007825 | Pcg3121-fbp | 4 | 0.12471 | 0.00974 | -84.679 |
| 7000007733 | Pcg0755-fbp | 4 | 0.08217 | 0.00974 | -89.9052 |
| 7000007746 | Pcg0755-hom | 8 | 0.81925 | 0.00689 | 0.647436 |
| 7000007792 | Pcg3381-hom | 4 | 0.77674 | 0.00974 | -4.57505 |
| 7000007723 | Pcg1860-hom | 8 | 0.71034 | 0.00689 | -12.7325 |
| 7000007838 | Pcg3121-hom | 8 | 0.559 | 0.00689 | -31.3251 |
| 7000007800 | Pcg0007_119-icd | 8 | 0.83236 | 0.00689 | 2.258041 |
| 7000007823 | Pcg3121-icd | 8 | 0.83155 | 0.00689 | 2.15853 |
| 7000007777 | Pcg3381-icd | 8 | 0.82844 | 0.00689 | 1.776456 |
| 7000007708 | Pcg1860-icd | 8 | 0.82384 | 0.00689 | 1.211332 |
| 7000007662 | Pcg0007_39-icd | 12 | 0.82008 | 0.00562 | 0.749404 |
| 7000007685 | Pcg0007-icd | 8 | 0.81257 | 0.00689 | -0.17322 |
| 7000007754 | Pcg0007_265-icd | 4 | 0.81172 | 0.00974 | -0.27765 |
| 7000007698 | Pcg0007-lysA | 4 | 0.8504 | 0.00974 | 4.474311 |
| 7000007675 | Pcg0007_39-lysA | 8 | 0.84414 | 0.00689 | 3.705251 |
| 7000007836 | Pcg3121-lysA | 4 | 0.83545 | 0.00974 | 2.637657 |
| 7000007767 | Pcg0007_265-lysA | 8 | 0.83249 | 0.00689 | 2.274012 |
| 7000007813 | Pcg0007_119-lysA | 8 | 0.83096 | 0.00689 | 2.086046 |
| 7000007790 | Pcg3381-lysA | 8 | 0.8118 | 0.00689 | -0.26782 |
| 7000007676 | Pcg0007_39-lysE | 8 | 0.84394 | 0.00689 | 3.68068 |
| 7000007699 | Pcg0007-lysE | 4 | 0.83393 | 0.00974 | 2.45092 |
| 7000007768 | Pcg0007_265-lysE | 8 | 0.83338 | 0.00689 | 2.383351 |
| 7000007837 | Pcg3121-lysE | 4 | 0.83199 | 0.00974 | 2.212585 |
| 7000007791 | Pcg3381-lysE | 8 | 0.81476 | 0.00689 | 0.095825 |
| 7000007814 | Pcg0007_119-lysE | 8 | 0.81315 | 0.00689 | -0.10197 |
| 7000007775 | Pcg3381-odx | 8 | 0.82237 | 0.00689 | 1.030738 |
| 7000007752 | Pcg0007_265-odx | 8 | 0.81118 | 0.00689 | -0.34399 |
| 7000007729 | Pcg0755-odx | 8 | 0.81103 | 0.00689 | -0.36242 |
| 7000007683 | Pcg0007-odx | 8 | 0.80507 | 0.00689 | -1.09462 |
| 7000007706 | Pcg1860-odx | 4 | 0.79332 | 0.00974 | -2.53815 |
| 7000007660 | Pcg0007_39-odx | 8 | 0.79149 | 0.00689 | -2.76297 |
| 7000007798 | Pcg0007_119-odx | 8 | 0.77075 | 0.00689 | -5.31094 |
| 7000007821 | Pcg3121-odx | 4 | 0.74788 | 0.00974 | -8.12059 |
| 7000007822 | Pcg3121-pck | 8 | 0.85544 | 0.00689 | 5.093491 |
| 7000007776 | Pcg3381-pck | 8 | 0.8419 | 0.00689 | 3.43006 |
| 7000007799 | Pcg0007_119-pck | 8 | 0.83851 | 0.00689 | 3.013588 |
| 7000007753 | Pcg0007_265-pck | 8 | 0.82738 | 0.00689 | 1.646232 |
| 7000007730 | Pcg0755-pck | 4 | 0.81785 | 0.00974 | 0.475442 |
| 7000007661 | Pcg0007_39-pck | 8 | 0.80976 | 0.00689 | -0.51844 |
| 7000007684 | Pcg0007-pck | 8 | 0.79007 | 0.00689 | -2.93742 |
| 7000007707 | Pcg1860-pck | 8 | 0.71566 | 0.00689 | -12.0789 |
| 7000007840 | Pcg3121-pgi | 4 | 1.01046 | 0.00974 | 24.13819 |
| 7000007817 | Pcg0007_119-pgi | 7 | 0.99238 | 0.00736 | 21.917 |
| 7000007794 | Pcg3381-pgi | 7 | 0.99008 | 0.00736 | 21.63444 |
| 7000007771 | Pcg0007_265-pgi | 8 | 0.94665 | 0.00689 | 16.29893 |
| 7000007725 | Pcg1860-pgi | 8 | 0.85515 | 0.00689 | 5.057864 |
| 7000007702 | Pcg0007-pgi | 4 | 0.8056 | 0.00974 | -1.02951 |
| 7000007658 | Pcg0007_39-ppc | 4 | 0.85221 | 0.00974 | 4.696676 |
| 7000007750 | Pcg0007_265-ppc | 8 | 0.84486 | 0.00689 | 3.793705 |
| 7000007727 | Pcg0755-ppc | 8 | 0.84166 | 0.00689 | 3.400575 |
| 7000007773 | Pcg3381-ppc | 4 | 0.82883 | 0.00974 | 1.824369 |
| 7000007796 | Pcg0007_119-ppc | 8 | 0.82433 | 0.00689 | 1.27153 |
| 7000007704 | Pcg1860-ppc | 8 | 0.81736 | 0.00689 | 0.415244 |
| 7000007819 | Pcg3121-ppc | 8 | 0.79898 | 0.00689 | -1.8428 |
| 7000007732 | Pcg0755-ptsG | 8 | 0.84055 | 0.00689 | 3.264208 |
| 7000007709 | Pcg1860-ptsG | 8 | 0.81075 | 0.00689 | -0.39682 |
| 7000007663 | Pcg0007_39-ptsG | 8 | 0.80065 | 0.00689 | -1.63763 |
| 7000007778 | Pcg3381-ptsG | 8 | 0.23419 | 0.00689 | -71.229 |
| 7000007801 | Pcg0007_119-ptsG | 8 | 0.17295 | 0.00689 | -78.7525 |
| 7000007824 | Pcg3121-ptsG | 8 | 0.16035 | 0.00689 | -80.3005 |
| 7000007705 | Pcg1860-pyc | 8 | 0.85143 | 0.00689 | 4.60085 |
| 7000007728 | Pcg0755-pyc | 8 | 0.79803 | 0.00689 | -1.95951 |
| 7000007659 | Pcg0007_39-pyc | 8 | 0.75539 | 0.00689 | -7.19797 |
| 7000007751 | Pcg0007_265-pyc | 8 | 0.73664 | 0.00689 | -9.50146 |
| 7000007682 | Pcg0007-pyc | 4 | 0.73142 | 0.00974 | -10.1428 |
| 7000007774 | Pcg3381-pyc | 4 | 0.66667 | 0.00974 | -18.0975 |
| 7000007797 | Pcg0007_119-pyc | 4 | 0.52498 | 0.00974 | -35.5046 |
| 7000007820 | Pcg3121-pyc | 8 | 0.52235 | 0.00689 | -35.8277 |
| 7000007841 | Pcg3121-tkt | 8 | 0.82565 | 0.00689 | 1.433696 |
| 7000007818 | Pcg0007_119-tkt | 8 | 0.81674 | 0.00689 | 0.339075 |
| 7000007749 | Pcg0755-tkt | 8 | 0.81496 | 0.00689 | 0.120396 |
| 7000007703 | Pcg0007-tkt | 4 | 0.76763 | 0.00974 | -5.69424 |
| 7000007795 | Pcg3381-tkt | 8 | 0.72213 | 0.00689 | -11.2841 |
| 7000007772 | Pcg0007_265-tkt | 8 | 0.68884 | 0.00689 | -15.3738 |
| 7000007701 | Pcg0007-zwf | 4 | 0.95061 | 0.00974 | 16.78542 |
| 7000007747 | Pcg0755-zwf | 8 | 0.92595 | 0.00689 | 13.75587 |
| 7000007770 | Pcg0007_265-zwf | 8 | 0.9029 | 0.00689 | 10.9241 |
| 7000007724 | Pcg1860-zwf | 8 | 0.79309 | 0.00689 | -2.5664 |
| 7000007839 | Pcg3121-zwf | 4 | 0.13379 | 0.00974 | -83.5635 |

### Example 4: Engineering the L-lysine biosynthetic pathway

The yield of L-lysine is modified by swapping pairs of promoters for target genes as provided in Table 8. The constructs of Example 3 are used to prepare recombinant organsims as provided in Table 8. As shown, the combination of Pcg0007-lysA and Pcg3121-pgi provide for the highest yields of L-lysine.

**Table 8: Paired Promoter Swapping of Target Genes in the L-lysine biosynthetic pathway**

| **Strain ID** | **Number** | **PRO Swap 1** | **PRO Swap 2** | **Mean Yield (A₅₆₀)** | **Std Dev** |
|---|---|---|---|---|---|
| 7000008489 | 4 | Pcg0007-lysA | Pcg3121-pgi | 1.17333 | 0.020121 |
| 7000008530 | 8 | Pcg1860-pyc | Pcg0007-zwf | 1.13144 | 0.030023 |
| 7000008491 | 7 | Pcg0007-lysA | Pcg0007-zwf | 1.09836 | 0.028609 |
| 7000008504 | 8 | Pcg3121-pck | Pcg0007-zwf | 1.09832 | 0.021939 |
| 7000008517 | 8 | Pcg0007_39-ppc | Pcg0007-zwf | 1.09502 | 0.030777 |
| 7000008502 | 4 | Pcg3121-pck | Pcg3121-pgi | 1.09366 | 0.075854 |
| 7000008478 | 4 | Pcg3381-ddh | Pcg0007-zwf | 1.08893 | 0.025505 |
| 7000008465 | 4 | Pcg0007_265-dapB | Pcg0007-zwf | 1.08617 | 0.025231 |
| 7000008535 | 8 | Pcg0007-zwf | Pcg3121-pgi | 1.06261 | 0.019757 |
| 7000008476 | 6 | Pcg3381-ddh | Pcg3121-pgi | 1.04808 | 0.084307 |
| 7000008510 | 8 | Pcg3121-pgi | Pcg1860-pyc | 1.04112 | 0.021087 |
| 7000008525 | 8 | Pcg1860-pyc | Pcg0007_265-dapB | 1.0319 | 0.034045 |
| 7000008527 | 8 | Pcg1860-pyc | Pcg0007-lysA | 1.02278 | 0.043549 |
| 7000008452 | 5 | Pcg1860-asd | Pcg0007-zwf | 1.02029 | 0.051663 |
| 7000008463 | 4 | Pcg0007_265-dapB | Pcg3121-pgi | 1.00511 | 0.031604 |
| 7000008524 | 8 | Pcg1860-pyc | Pcg1860-asd | 1.00092 | 0.026355 |
| 7000008458 | 4 | Pcg3381-aspB | Pcg1860-pyc | 1.00043 | 0.020083 |
| 7000008484 | 8 | Pcg3381-fbp | Pcg1860-pyc | 0.99686 | 0.061364 |
| 7000008474 | 8 | Pcg3381-ddh | Pcg3381-fbp | 0.99628 | 0.019733 |
| 7000008522 | 8 | Pcg0755-ptsG | Pcg3121-pgi | 0.99298 | 0.066021 |
| 7000008528 | 8 | Pcg1860-pyc | Pcg3121-pck | 0.99129 | 0.021561 |
| 7000008450 | 4 | Pcg1860-asd | Pcg3121-pgi | 0.98262 | 0.003107 |
| 7000008448 | 8 | Pcg1860-asd | Pcg3381-fbp | 0.97814 | 0.022285 |
| 7000008494 | 8 | Pcg0007 _39-lysE | Pcg3381-fbp | 0.97407 | 0.027018 |
| 7000008481 | 8 | Pcg3381-fbp | Pcg0007-lysA | 0.9694 | 0.029315 |
| 7000008497 | 8 | Pcg0007 _39-lysE | Pcg1860-pyc | 0.9678 | 0.028569 |
| 7000008507 | 8 | Pcg3121-pgi | Pcg3381-fbp | 0.96358 | 0.035078 |
| 7000008501 | 8 | Pcg3121-pck | Pcg0007-lysA | 0.96144 | 0.018665 |
| 7000008486 | 8 | Pcg0007-lysA | Pcg0007_265-dapB | 0.94523 | 0.017578 |
| 7000008459 | 8 | Pcg0007_265-dapB | Pcg1860-asd | 0.94462 | 0.023847 |
| 7000008506 | 2 | Pcg3121-pgi | Pcg0007_265-dapD | 0.94345 | 0.014014 |
| 7000008487 | 8 | Pcg0007-lysA | Pcg3381-ddh | 0.94249 | 0.009684 |
| 7000008498 | 8 | Pcg3121-pck | Pcg1860-asd | 0.94154 | 0.016802 |
| 7000008485 | 8 | Pcg0007-lysA | Pcg1860-asd | 0.94135 | 0.013578 |
| 7000008499 | 8 | Pcg3121-pck | Pcg0007_265-dapB | 0.93805 | 0.013317 |
| 7000008472 | 8 | Pcg3381-ddh | Pcg1860-asd | 0.93716 | 0.012472 |
| 7000008511 | 8 | Pcg0007_39-ppc | Pcg1860-asd | 0.93673 | 0.015697 |
| 7000008514 | 8 | Pcg0007_39-ppc | Pcg0007-lysA | 0.93668 | 0.027204 |
| 7000008473 | 8 | Pcg3381-ddh | Pcg0007_265-dapB | 0.93582 | 0.030377 |
| 7000008461 | 7 | Pcg0007_265-dapB | Pcg3381-fbp | 0.93498 | 0.037862 |
| 7000008512 | 8 | Pcg0007_39-ppc | Pcg0007_265-dapB | 0.93033 | 0.017521 |
| 7000008456 | 8 | Pcg3381-aspB | Pcg3121-pck | 0.92544 | 0.020075 |
| 7000008460 | 8 | Pcg0007_265-dapB | Pcg0007_265-dapD | 0.91723 | 0.009508 |
| 7000008492 | 8 | Pcg0007 _39-lysE | Pcg3381-aspB | 0.91165 | 0.012988 |
| 7000008493 | 8 | Pcg0007 _39-lysE | Pcg0007_265-dapD | 0.90609 | 0.031968 |
| 7000008453 | 8 | Pcg3381-aspB | Pcg0007_265-dapB | 0.90338 | 0.013228 |
| 7000008447 | 8 | Pcg1860-asd | Pcg0007_265-dapD | 0.89886 | 0.028896 |
| 7000008455 | 8 | Pcg3381-aspB | Pcg0007-lysA | 0.89531 | 0.027108 |
| 7000008454 | 6 | Pcg3381-aspB | Pcg3381-ddh | 0.87816 | 0.025807 |
| 7000008523 | 8 | Pcg0755-ptsG | Pcg1860-pyc | 0.87693 | 0.030322 |
| 7000008520 | 8 | Pcg0755-ptsG | Pcg3381-fbp | 0.87656 | 0.018452 |
| 7000008533 | 4 | Pcg0007-zwf | Pcg3381-fbp | 0.84584 | 0.017012 |
| 7000008519 | 8 | Pcg0755-ptsG | Pcg0007_265-dapD | 0.84196 | 0.025747 |

## Claims

1. A promoter ladder comprising at least three promoter polynucleotide sequences selected from the group consisting of SEQ ID NOs: 1 to 8, wherein said promoter ladder comprises a plurality of promoters with incrementally increasing levels of promoter activity.

2. The promoter ladder according to claim 1, wherein at least one of said promoter polynucleotide sequences is selected from the group consisting of SEQ ID NOs: 1, 5 and 7.

3. The promoter ladder according to claim 1, wherein at least one of said promoter polynucleotide sequences is selected from the group consisting of SEQ ID NOs: 1-2.

4. The promoter ladder according to claim 1, wherein at least one of said promoter polynucleotide sequences is selected from the group consisting of SEQ ID NOs: 3, 4 and 5.

5. The promoter ladder according to claim 1, wherein at least one of said promoter polynucleotide sequences is selected from the group consisting of SEQ ID NOs: 6-8.

6. The promoter ladder according to any one of claims 1-5, wherein each promoter of said promoter ladder is functionally linked to at least one heterologous target gene.

7. The promoter ladder according to claim 6, wherein said at least one heterologous target gene is a gene that is a component of a biosynthetic pathway producing a biomolecule selected from the group consisting of amino acids, organic acids, proteins and polymers.

8. The promoter ladder according to claim 6 or 7, wherein said at least one heterologous target gene is a gene that is a component of an amino acid biosynthetic pathway selected from the group consisting of:
- the lysine biosynthesis pathway comprising genes of entry M00016 of the Kyoto Encyclopedia of Genes and Genomes (KEGG);
- the lysine biosynthesis pathway comprising genes of KEGG entry M00525;
- the lysine biosynthesis pathway comprising genes of KEGG entry M00526;
- the lysine biosynthesis pathway comprising genes of KEGG entry M00527;
- the lysine biosynthesis pathway comprising genes of KEGG entry M0030;
- the lysine biosynthesis pathway comprising genes of KEGG entry M00433; and the lysine biosynthesis pathway comprising genes of KEGG entry M0031.

9. A recombinant polynucleotide comprising a promoter polynucleotide sequence selected from the promoter ladder of claim 1 functionally linked to a heterologous target gene.

10. A plurality of recombinant polynucleotides comprising the promoter ladder of claim 1, wherein each recombinant polynucleotide comprises one promoter from the promoter ladder functionally linked to a heterologous target gene.

11. A plurality of recombinant vectors comprising the promoter ladder of any one of claims 1-8, the recombinant polynucleotide of claim 9, or the plurality of recombinant polynucleotides of claim 10, wherein each vector comprises at least one promoter from the promoter ladder or at least one recombinant polynucleotide.

12. A plurality of host cells comprising the promoter ladder according to any one of claims 1-8, the recombinant polynucleotide of claim 9, the plurality of recombinant polynucleotides of claim 10, or the plurality of recombinant vectors of claim 11, wherein each host cell comprises at least one promoter from the promoter ladder, at least one recombinant polynucleotide, or at least one vector.

13. The plurality of host cells according to claim 12, wherein the host cells belong to the genus *Corynebacterium.*

14. Transformed host cells comprising a combination of at least three promoter polynucleotide sequences selected from the group consisting of SEQ ID Nos: 1 to 8 each functionally linked to at least one heterologous target gene, wherein said combination of promoter polynucleotides comprises a plurality of promoters with incrementally increasing levels of promoter activity.

15. The transformed host cells according to claim 14, wherein said target gene is associated with a biosynthetic pathway producing a biomolecule selected from: amino acids, organic acids, flavors and fragrances, biofuels, proteins and enzymes, polymers/monomers and other biomaterials, lipids, nucleic acids, small molecule therapeutics, protein therapeutics, fine chemicals, and nutraceuticals.

16. The transformed host cells according to claim 15, wherein said target gene is associated with a biosynthetic pathway producing a secondary metabolite selected from:
antibiotics, alkaloids, terpenoids, and polyketides.

17. The transformed host cells according to any one of claims 14-16, wherein each promoter polynucleotide is functionally linked to a different heterologous target gene.

## Patentansprüche

1. Promotorenleiter, die mindestens drei Promotor-Polynukleotidsequenzen umfasst, die aus der Gruppe bestehend aus den SEQ ID Nrn. 1 bis 8 ausgewählt sind, wobei die Promotorenleiter mehrere Promotoren mit stufenweise ansteigenden Graden an Promotor-Aktivität umfasst.

2. Promotorenleiter nach Anspruch 1, wobei mindestens eine der Promotor-Polynukleotidsequenzen aus der Gruppe bestehend aus den SEQ ID Nrn. 1, 5 und 7 ausgewählt ist.

3. Promotorenleiter nach Anspruch 1, wobei mindestens eine der Promotor-Polynukleotidsequenzen aus der Gruppe bestehend aus den SEQ ID Nrn. 1-2 ausgewählt ist.

4. Promotorenleiter nach Anspruch 1, wobei mindestens eine der Promotor-Polynukleotidsequenzen aus der Gruppe bestehend aus den SEQ ID Nrn. 3, 4 und 5 ausgewählt ist.

5. Promotorenleiter nach Anspruch 1, wobei mindestens eine der Promotor-Polynukleotidsequenzen aus der Gruppe bestehend aus den SEQ ID Nrn. 6-8 ausgewählt ist.

6. Promotorenleiter nach einem der Ansprüche 1-5, wobei jeder Promotor der Promotorenleiter mit mindestens einem heterologen Zielgen funktionell verknüpft ist.

7. Promotorenleiter nach Anspruch 6, wobei das mindestens eine heterologe Zielgen ein Gen ist, das eine Komponente eines biosynthetischen Wegs ist, der ein Biomolekül produziert, das aus der Gruppe bestehend aus Aminosäuren, organischen Säuren, Proteinen und Polymeren ausgewählt ist.

8. Promotorenleiter nach Anspruch 6 oder 7, wobei das mindestens eine heterologe Zielgen ein Gen ist, das eine Komponente eines biosynthetischen Aminosäure-Wegs ist, der ausgewählt ist aus der Gruppe bestehend aus:
- dem Lysin-Biosyntheseweg, der Gene von Eintrag M00016 der Kyoto Encyclopedia of Genes and Genomes (KEGG) umfasst;
- dem Lysin-Biosyntheseweg, der Gene von KEGG-Eintrag M00525 umfasst;
- dem Lysin-Biosyntheseweg, der Gene von KEGG-Eintrag M00526 umfasst;
- dem Lysin-Biosyntheseweg, der Gene von KEGG-Eintrag M00527 umfasst;
- dem Lysin-Biosyntheseweg, der Gene von KEGG-Eintrag M0030 umfasst;
- dem Lysin-Biosyntheseweg, der Gene von KEGG-Eintrag M00433 umfasst; und dem Lysin-Biosyntheseweg, der Gene von KEGG-Eintrag M0031 umfasst.

9. Rekombinantes Polynukleotid, das eine Promotor-Polynukleotidsequenz umfasst, die aus der Promotorenleiter nach Anspruch 1 ausgewählt ist, die mit einem heterologen Zielgen funktionell verknüpft ist.

10. Mehrere rekombinante Polynukleotide, die die Promotorenleiter nach Anspruch 1 umfassen, wobei jedes rekombinante Polynukleotid einen Promotor aus der Promotorenleiter umfasst, der mit einem heterologen Zielgen funktionell verknüpft ist.

11. Mehrere rekombinante Vektoren, die die Promotorenleiter nach einem der Ansprüche 1-8, das rekombinante Polynukleotid nach Anspruch 9 oder die mehreren rekombinanten Polynukleotide nach Anspruch 10 umfassen, wobei jeder Vektor mindestens einen Promotor aus der Promotorenleiter oder mindestens ein rekombinantes Polynukleotid umfasst.

12. Mehrere Wirtszellen, die die Promotorenleiter nach einem der Ansprüche 1-8, das rekombinante Polynukleotid nach Anspruch 9, die mehreren rekombinanten Polynukleotide nach Anspruch 10 oder die mehreren rekombinanten Vektoren nach Anspruch 11 umfassen, wobei jede Wirtszelle mindestens einen Promotor aus der Promotorenleiter, mindestens ein rekombinantes Polynukleotid oder mindestens einen Vektor umfasst.

13. Mehrere Wirtszellen nach Anspruch 12, wobei die Wirtszellen zur Gattung *Corynebacterium* gehören.

14. Transformierte Wirtszellen, die eine Kombination von mindestens drei Promotor-Polynukleotidsequenzen umfassen, die aus der Gruppe bestehend aus den SEQ ID Nrn. 1 bis 8 ausgewählt sind und die jeweils mit mindestens einem heterologen Zielgen funktionell verknüpft sind, wobei die Kombination von Promotor-Polynukleotiden mehrere Promotoren mit stufenweise ansteigenden Graden an Promotor-Aktivität umfasst.

15. Transformierte Wirtszellen nach Anspruch 14, wobei das Zielgen mit einem biosynthetischen Weg assoziiert ist, der ein Biomolekül produziert, das ausgewählt ist aus: Aminosäuren, organischen Säuren, Geschmackstoffen und Duftstoffen, Biotreibstoffen, Proteinen und Enzymen, Polymeren/Monomeren und anderen Biomaterialien, Lipiden, Nukleinsäuren, Kleinmolekül-Therapeutika, Protein-Therapeutika, Feinchemikalien und Nutrazeutika.

16. Transformierte Wirtszellen nach Anspruch 15, wobei das Zielgen mit einem biosynthetischen Weg assoziiert ist, der einen sekundären Metaboliten produziert, der ausgewählt ist aus: Antibiotika, Alkaloiden, Terpenoiden und Polyketiden.

17. Transformierte Wirtszellen nach einem der Ansprüche 14-16, wobei jedes Promotor-Polynukleotid mit einem anderen heterologen Zielgen funktionell verknüpft ist.

## Revendications

1. Échelle de promoteurs comprenant au moins trois séquences polynucléotidiques promotrices choisies dans le groupe constitué par les SEQ ID n^{os} : 1 à 8, ladite échelle de promoteurs comprenant une pluralité de promoteurs présentant des niveaux progressivement croissants d'activité de promoteur.

2. Échelle de promoteurs selon la revendication 1, dans laquelle au moins l'une desdites séquences polynucléotidiques promotrices est choisie dans le groupe constitué par les SEQ ID n^{os} : 1, 5 et 7.

3. Échelle de promoteurs selon la revendication 1, dans laquelle au moins l'une desdites séquences polynucléotidiques promotrices est choisie dans le groupe constitué par les SEQ ID n^{os} : 1-2.

4. Échelle de promoteurs selon la revendication 1, dans laquelle au moins l'une desdites séquences polynucléotidiques promotrices est choisie dans le groupe constitué par les SEQ ID n^{os} : 3, 4 et 5.

5. Échelle de promoteurs selon la revendication 1, dans laquelle au moins l'une desdites séquences polynucléotidiques promotrices est choisie dans le groupe constitué par les SEQ ID n^{os} : 6-8.

6. Échelle de promoteurs selon l'une quelconque des revendications 1-5, chaque promoteur de ladite échelle de promoteurs étant lié de manière fonctionnelle à au moins un gène cible hétérologue.

7. Échelle de promoteurs selon la revendication 6, dans laquelle ledit au moins un gène cible hétérologue est un gène qui est un composant d'une voie biosynthétique produisant une biomolécule choisie dans le groupe constitué par les acides aminés, les acides organiques, les protéines et les polymères.

8. Échelle de promoteurs selon la revendication 6 ou la revendication 7, dans laquelle ledit au moins un gène cible hétérologue est un gène qui est un composant d'une voie biosynthétique d'acide aminé choisie dans le groupe constitué par :
- la voie de biosynthèse de lysine comprenant des gènes de l'entrée de l'Encyclopédie de Kyoto des gènes et des génomes (KEGG) M00016 ;
- la voie de biosynthèse de lysine comprenant des gènes de l'entrée KEGG M00525 ;
- la voie de biosynthèse de lysine comprenant des gènes de l'entrée KEGG M00526 ;
- la voie de biosynthèse de lysine comprenant des gènes de l'entrée KEGG M00527 ;
- la voie de biosynthèse de lysine comprenant des gènes de l'entrée KEGG M0030 ;
- la voie de biosynthèse de lysine comprenant des gènes de l'entrée KEGG M00433 ; et
- la voie de biosynthèse de lysine comprenant des gènes de l'entrée KEGG M0031.

9. Polynucléotide recombiné comprenant une séquence polynucléotidique promotrice choisie dans l'échelle de promoteurs de la revendication 1 liée de manière fonctionnelle à un gène cible hétérologue.

10. Pluralité de polynucléotides recombinés comprenant l'échelle de promoteurs de la revendication 1, dans laquelle chaque polynucléotide recombiné comprend un promoteur provenant de l'échelle de promoteurs lié de manière fonctionnelle à un gène cible hétérologue.

11. Pluralité de vecteurs recombinés comprenant l'échelle de promoteurs de l'une quelconque des revendications 1-8, le polynucléotide recombiné de la revendication 9 ou la pluralité de polynucléotides recombinés de la revendication 10, dans laquelle chaque vecteur comprend au moins un promoteur provenant de l'échelle de promoteurs ou au moins un polynucléotide recombiné.

12. Pluralité de cellules hôtes comprenant l'échelle de promoteurs de l'une quelconque des revendications 1-8, le polynucléotide recombiné de la revendication 9, la pluralité de polynucléotides recombinés de la revendication 10 ou la pluralité de vecteurs recombinés de la revendication 11, dans laquelle chaque cellule hôte comprend au moins un promoteur provenant de l'échelle de promoteurs, au moins un polynucléotide recombiné ou au moins un vecteur.

13. Pluralité de cellules hôtes selon la revendication 12, dans laquelle les cellules hôtes appartiennent au genre *Corynebacterium.*

14. Cellules hôtes transformées comprenant une combinaison d'au moins trois séquences polynucléotidiques promotrices choisies dans le groupe constitué par les SEQ ID n^{os} : 1 à 8 chacune liée de manière fonctionnelle à au moins un gène cible hétérologue, dans lesquelles ladite combinaison de polynucléotides promoteurs comprend une pluralité de promoteurs présentant des niveaux progressivement croissants d'activité de promoteur.

15. Cellules hôtes transformées selon la revendication 14, dans lesquelles ledit gène cible est associé à une voie biosynthétique produisant une biomolécule choisie parmi : des acides aminés, des acides organiques, des arômes et fragrances, des biocarburants, des protéines et enzymes, des polymères/monomères et autres biomatériaux, des lipides, des acides nucléiques, des agents thérapeutiques à petite molécule, des agents thérapeutiques protéiques, des produits de chimie fine et des produits nutraceutiques.

16. Cellules hôtes transformées selon la revendication 15, dans lesquelles ledit gène cible est associé à une voie biosynthétique produisant un métabolite secondaire choisi parmi : des antibiotiques, des alcaloïdes, des terpénoïdes et des polycétides.

17. Cellules hôtes transformées selon l'une quelconque des revendications 14-16, dans lesquelles chaque polynucléotide promoteur est lié de manière fonctionnelle à un gène cible hétérologue différent.


<110> ZYMERGEN, INC.
<120> PROMOTERS FROM CORYNEBACTERIUM GLUTAMICUM
<130> ZMG-001/PCT
<140>
   <141>
<150> 62/264,232
   <151> 2015-12-07
<160> 16
<170> PatentIn version 3.5
<210> 1
   <211> 97
   <212> DNA
   <213> Corynebacterium glutamicum
<220>
   <221> source
   <223> /note="Pcg0007_39"
<400> 1
   tgccgtttct cgcgttgtgt gtggtactac gtggggacct aagcgtgtat tatggaaacg       60
   tctgtatcgg ataagtagcg aggagtgttc gttaaaa                                97
<210> 2
   <211> 97
   <212> DNA
   <213> Corynebacterium glutamicum
<220>
   <221> source
   <223> /note="Pcg0007"
<400> 2
   tgccgtttct cgcgttgtgt gtggtactac gtggggacct aagcgtgtaa gatggaaacg 60
   tctgtatcgg ataagtagcg aggagtgttc gttaaaa 97
<210> 3
   <211> 93
   <212> DNA
   <213> Corynebacterium glutamicum
<220>
   <221> source
   <223> /note="Pcg1860"
<400> 3
   cttagctttg acctgcacaa atagttgcaa attgtcccac atacacataa agtagcttgc 60
   gtatttaaaa ttatgaacct aaggggttta gca 93
<210> 4
   <211> 98
   <212> DNA
   <213> Corynebacterium glutamicum
<220>
   <221> source
   <223> /note="Pcg0755"
<400> 4
   aataaattta taccacacag tctattgcaa tagaccaagc tgttcagtag ggtgcatggg 60
   agaagaattt cctaataaaa actcttaagg acctccaa 98
<210> 5
   <211> 97
   <212> DNA
   <213> Corynebacterium glutamicum
<220>
   <221> source
   <223> /note="Pcg0007_265"
<400> 5
   tgccgtttct cgcgttgtgt gtggtactac gtggggacct aagcgtgtac gctggaaacg 60
   tctgtatcgg ataagtagcg aggagtgttc gttaaaa 97
<210> 6
   <211> 86
   <212> DNA
   <213> Corynebacterium glutamicum
<220>
   <221> source
   <223> /note="Pcg3381"
<400> 6
   cgccggataa atgaattgat tattttaggc tcccagggat taagtctagg gtggaatgca 60
   gaaatatttc ctacggaagg tccgtt 86
<210> 7
   <211> 97
   <212> DNA
   <213> Corynebacterium glutamicum
<220>
   <221> source
   <223> /note="Pcg0007_119"
<400> 7
   tgccgtttct cgcgttgtgt gtggtactac gtggggacct aagcgtgttg catggaaacg 60
   tctgtatcgg ataagtagcg aggagtgttc gttaaaa 97
<210> 8
   <211> 87
   <212> DNA
   <213> Corynebacterium glutamicum
<220>
   <221> source
   <223> /note="Pcg3121"
<400> 8
   gtggctaaaa cttttggaaa cttaagttac ctttaatcgg aaacttattg aattcgggtg 60
   aggcaactgc aactctggac ttaaagc 87
<210> 9
   <211> 24
   <212> DNA
   <213> Artificial Sequence
<220>
   <221> source
   <223> /note="Description of Artificial Sequence: Synthetic primer"
<400> 9
   tcatgaccaa aatcccttaa cgtg 24
<210> 10
   <211> 28
   <212> DNA
   <213> Artificial Sequence
<220>
   <221> source
   <223> /note="Description of Artificial Sequence: Synthetic primer"
<400> 10
   gcgtactctt cgatggtgaa aacatctc 28
<210> 11
   <211> 57
   <212> DNA
   <213> Artificial Sequence
<220>
   <221> source
   <223> /note="Description of Artificial Sequence: Synthetic primer"
<400> 11
   gacctaaaat gtgtaaaggg caaagtgtat acaacaacaa gacccatcat agtttgc 57
<210> 12
   <211> 44
   <212> DNA
   <213> Artificial Sequence
<220>
   <221> source
   <223> /note="Description of Artificial Sequence: Synthetic primer"
<400> 12
   cacgttaagg gattttggtc atgacacatg cagtcatgtc gtgc 44
<210> 13
   <211> 21
   <212> DNA
   <213> Artificial Sequence
<220>
   <221> source
   <223> /note="Description of Artificial Sequence: Synthetic primer"
<400> 13
   gcttgcatgc ctgcaggtcg a 21
<210> 14
   <211> 22
   <212> DNA
   <213> Artificial Sequence
<220>
   <221> source
   <223> /note="Description of Artificial Sequence: Synthetic primer"
<400> 14
   atggtgagca agggcgagga gc 22
<210> 15
   <211> 25
   <212> DNA
   <213> Artificial Sequence
<220>
   <221> source
   <223> /note="Description of Artificial Sequence: Synthetic primer"
<400> 15
   ggaaacgtct gtatcggata agtag 25
<210> 16
   <211> 57
   <212> DNA
   <213> Artificial Sequence
<220>
   <221> source
   <223> /note="Description of Artificial Sequence: Synthetic primer"
<220>
   <221> modified_base
   <222> (27)..(30)
   <223> a, c, t, g, unknown or other
<400> 16
   ctacttatcc gatacagacg tttccannnn acacgcttag gtccccacgt agtacca 57